# EUROPEAN PATENT SPECIFICATION

(11) **EP 2 429 708 B1**
(45) Date of publication and mention of the grant of the patent: **30.12.2020**
(21) Application number: 10719712.1
(22) Date of filing: 14.05.2010
(51) Int. Cl.: B01L 3/14

(54) **DENSITY PHASE SEPARATION DEVICE**
DICHTE-PHASENTRENNVORRICHTUNG
DISPOSITIF DE SEPARATION DE PHASES PAR DENSITE

(30) Priority: 15.05.2009 US 178599 P
(43) Date of publication of application: 21.03.2012
(60) Divisional application: 15159646.7 / 2 913 108; 15159653.3 / 2 915 586; 15159656.6 / 3 006 109; 15159679.8 / 2 918 344; 15159681.4 / 2 923 765; 15159704.4 / 2 921 233
(73) Proprietor: Becton, Dickinson and Company, Franklin Lakes, NJ 07417-1880 (US)
(72) Inventor: CRAWFORD, Jamieson, W., Demarest New Jersey 07627 (US); ATTRI, Ravi, Budd Lake, New Jersey 07828 (US); BATTLES, Christopher, A., Seymour Connecticut 06483 (US); HIRES, Gregory, R., Fairfield Connecticut 06824 (US); BARTFELD, Benjamin, Ringwood New Jersey 07456 (US)
(74) Representative: dompatent von Kreisler Selting Werner - Partnerschaft von Patent- und Rechtsanwälten mbB
(86) International application number: PCT/US2010/034920
(87) International publication number: WO 2010/132783

(56) References cited:
- EP-A2- 1 106 252
- EP-A2- 1 106 253
- US-A- 4 088 582
- US-A- 4 364 832
- US-A- 5 632 905
- US-A1- 2002 132 367

## Description

### BACKGROUND OF THE INVENTION

### Field of the Invention

The subject invention relates to a device for separating higher and lower density fractions of a fluid sample. More particularly, this invention relates to a device for collecting and transporting fluid samples whereby the device and fluid sample are subjected to centrifugation in order to cause separation of the higher density fraction from the lower density fraction of the fluid sample.

### Description of Related Art

Diagnostic tests may require separation of a patient's whole blood sample into components, such as serum or plasma (the lower density phase components), and red blood cells (the higher density phase components). Samples of whole blood are typically collected by venipuncture through a cannula or needle attached to a syringe or an evacuated blood collection tube. After collection, separation of the blood into serum or plasma and red blood cells is accomplished by rotation of the syringe or tube in a centrifuge. In order to maintain the separation, a barrier must be positioned between the higher density and lower density phase components. This allows the separated components to be subsequently examined.

A variety of separation barriers have been used in collection devices to divide the area between the higher density and lower density phases of a fluid sample. Examples can be found in EP 1 106 252 A2, US 4 088 582 A, US 5 632 905 A, US 4 364 832 A, EP 1 106 253 A2. The most widely used devices include thixotropic gel materials, such as polyester gels. However, current polyester gel serum separation tubes require special manufacturing equipment to both prepare the gel and fill the tubes. Moreover, the shelf-life of the gel-based separator product is limited. Over time, globules may be released from the gel mass and enter one or both of the separated phase components. Furthermore, commercially available gel barriers may react chemically with the analytes. Accordingly, if certain drugs are present in the blood sample when it is taken, an adverse chemical reaction with the gel interface can occur. Furthermore, if an instrument probe is inserted too deeply into a collection container, then the instrument probe may become clogged if it contacts the gel.

Certain mechanical separators have also been proposed in which a mechanical barrier can be employed between the higher and lower density phases of the fluid sample. Conventional mechanical barriers are positioned between higher and lower density phase components utilizing elevated gravitational forces applied during centrifugation. For proper orientation with respect to plasma and serum specimens, conventional mechanical separators are typically positioned above the collected whole blood specimen prior to centrifugation. This typically requires that the mechanical separator be affixed to the underside of the tube closure in such a manner that blood fill occurs through or around the device when engaged with a blood collection set or phlebotomy needle. This attachment is required to prevent the premature movement of the separator during shipment, handling, and blood draw. Conventional mechanical separators are typically affixed to the tube closure by a mechanical interlock between the bellows component and the closure.

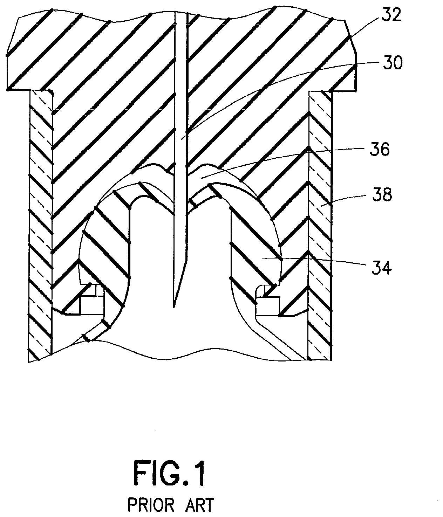

Conventional mechanical separators have some significant drawbacks. As shown in **FIG. 1****,** conventional separators include a bellows **34** for providing a seal with the tube or syringe wall **38.** Typically, at least a portion of the bellows **34** is housed within, or in contact with a closure **32.** As shown in **FIG. 1****,** as the needle **30** enters through the closure **32,** the bellows **34** is depressed. This creates a void **36** in which blood may pool during insertion or removal of the needle. This can result in sample pooling under the closure, device pre-launch in which the mechanical separator prematurely releases during blood collection, trapping of a significant quantity of fluid phases, such as serum and plasma, poor sample quality, and/or barrier failure under certain circumstances. Furthermore, previous mechanical separators are costly and complicated to manufacture due to the complicated multi-part fabrication techniques.

Accordingly, a need exists for a separator device that is compatible with standard sampling equipment and reduces or eliminates the aforementioned problems of conventional separators. A need also exists for a separator device that is easily used to separate a blood sample, minimizes cross-contamination of the higher and lower density phases of the sample during centrifugation, is independent of temperature during storage and shipping, and is stable to radiation sterilization. A need further exists for a unitary separation device that requires fewer relative moving parts and that allows for enhanced ease of introducing a specimen into a collection container.

### SUMMARY OF THE INVENTION

The present invention is directed to an assembly for separating a fluid sample into a higher density and a lower density phase as defined in claim 1. Desirably, the mechanical separator of the present invention may be used with a collection container, such as a tube, and is structured to move within the tube under the action of applied centrifugal force in order to separate the portions of a fluid sample. In certain configurations, the tube is a specimen collection tube including an open end, a closed end, and a sidewall extending between the open end and closed end. The sidewall includes an outer surface and an inner surface and the tube further includes a closure disposed to fit in the open end of the tube with a resealable septum. Alternatively, both ends of the tube may be open, and both ends of the tube may be sealed by elastomeric closures. At least one of the closures of the tube may include a needle pierceable resealable septum.

The mechanical separator may be disposed within the tube at a location between the top closure and the bottom of the tube. The components of the separator are dimensioned and configured to achieve an overall density for the separator that lies between the densities of the phases of a fluid sample, such as the higher and lower density phases of a blood sample.

In accordance with an embodiment of the present invention, a mechanical separator for separating a fluid sample into first and second phases within a collection container includes a separator body having a through-hole defined therein. The through-hole is adapted for allowing fluid to pass therethrough. The separator body includes a float, having a first density, and a ballast, having a second density greater than the first density. A portion of the float is connected to a portion of the ballast.

The mechanical separator may have a spheroid shape. Optionally, the float may include an exterior surface and a joining surface, and the ballast may include a contact surface connected to the joining surface of the float and an exterior surface. The exterior surface of the float and the exterior surface of the ballast taken together may form the spheroid shape.

In certain configurations, the float defines the through-hole adapted for allowing fluid to pass therethrough. The through-hole may have a circular cross-section. In other configurations, the through-hole may have an elliptical cross-section. The through-hole may be defined along a through-axis, and the float may be adapted for deformation in a direction perpendicular to the through-axis upon applied rotational force.

In another configuration, the float further includes a first extended tab adjacent a first opening of the through-hole and a second extended tab adjacent the second opening of the through-hole. At least a portion of the first extended tab and at least a portion of the second extended tab may be provided above and about the through-hole and extend radially outwardly from the float in a direction parallel to the through-axis of the separator body. Optionally, the first extended tab, an upper surface of the float, and the second extended tab may form a convex upper float surface.

In another configuration, the separator body further includes an extended tab band disposed about a portion of an outer surface of the float. Optionally, a first portion of the extended tab band is disposed adjacent a first opening of the through-hole, and a second portion of the extended tab band is disposed adjacent a second opening of the through-hole. In a further configuration, at least one of the first portion and the second portion of the extended tab band have a concave downwardly-directed orientation. Optionally, at least one of the first portion and the second portion of the extended tab band are oriented in an outwardly-extending arcuate shape about an upper portion of at least one of the first opening and second opening of the through-hole. At least one of the first portion and the second portion of the extended tab band may extend outwardly from the float in a direction parallel to the through-axis. At least a portion of the first extended portion and at least a portion of the second extended portion of the extended tab band may have the same shape and curvature. In certain configurations, the extended tab band may further include a joining portion disposed between and connecting the first extended portion and the second extended portion disposed on each connecting side of the separator body. The first extended portion and the second extended portion of the extended tab band have a concave downwardly-directed orientation, and the joining portions of the extended tab band have a concave upwardly-directed orientation. In certain configurations, the float may include the extended tab band. Optionally, the float and the extended tab band may be formed of TPE and the ballast is formed of PET.

The mechanical separator may also include an initial engagement band circumferentially disposed about the separator body. The initial engagement band may be continuous or at least partially segmented. The initial engagement band and the float may be formed of the same material. The initial engagement band may bisect at least a portion of the ballast.

In another configuration, the ballast may include a base portion and a joining structure for engaging a portion of the float. The joining structure may include a plurality of arms for engaging a portion of the float, and the joining structure may provide flexure between the float and the ballast. Optionally, at least a portion of the float may have a circular outer perimeter having a curved cross-section perpendicular to the through-hole. In certain configurations, the float may include a joining structure for engaging a portion of the ballast. The joining structure may include a plurality of arms for engaging a portion of the ballast, and the joining structure may provide flexure between the float and the ballast.

In accordance with another embodiment of the present invention, a separation assembly for enabling separation of a fluid sample into first and second phases includes a collection container having a first end, a second end, and a sidewall extending therebetween. The collection container defines a longitudinal axis between the first end and the second end. The separation assembly further includes a mechanical separator having a separator body having a through-hole defined therein. The separator body is adapted to transition from a first initial position in which the through-hole is oriented in an open position for allowing fluid to pass therethrough, to a second sealing position in which the through-hole is oriented in a closed position for preventing fluid from being received therethrough, upon applied rotational force.

In one configuration, the separation assembly further includes a closure adapted for sealing engagement with the first end of the collection container, with the mechanical separator releasably engaged with a portion of the closure. The mechanical separator may be engaged with a portion of the closure in the first initial position, and the mechanical separator may be engaged with a portion of the sidewall of the collection container in the second sealing position. The closure may include an engagement boss disposed within a portion of the through-hole when the separator body is in the first initial position for forming a fluid seal between a portion of the separator body and the closure. Optionally, at least a portion of the through-hole of the mechanical separator is oriented along the longitudinal axis of the collection container in the first initial position, and the through-hole is oriented perpendicular to the longitudinal axis of the collection container in the second sealing position. Transition of the through-hole from the open position to the closed position may coincide with rotation of the mechanical separator from the first initial position to the second sealing position. The mechanical separator may sealingly engage a portion of the collection container wall in the second sealing position to prevent flow of fluid therethrough or therearound.

In certain configurations, the separator body further includes a first extended tab adjacent a first opening of the through-hole and a second extended tab adjacent the second opening of the through-hole. The first extended tab and the second extended tab may engage a portion of the sidewall of the collection container in the second sealing position. In other configurations, the separator body further includes an extended tab band disposed about a portion of an outer surface of the float. The extended tab band may engage a portion of the sidewall of the collection container in the second sealing position, and the extended tab band may form a continuous seal with the sidewall of the collection container in the second sealing position.

In other configurations, the ballast includes a joining structure for engaging a portion of the float, and at least a portion of the float includes a circular outer perimeter having a curved cross-section perpendicular to the through-hole. The outer perimeter of the float may form a continuous seal with the sidewall of the collection container in the second sealing position. Optionally, the float includes a joining structure for engaging a portion of the ballast, and at least a portion of the float includes a circular outer perimeter having a curved cross-section perpendicular to the through-hole, with the outer perimeter of the float forming a continuous seal with the sidewall of the collection container in the second sealing position.

In accordance with another embodiment of the present invention, a separation assembly for enabling separation of a fluid sample into first and second phases includes a collection container having a first end, a second end, and a sidewall extending therebetween. The separation assembly further includes a mechanical separator having a separator body having a through-hole defined therein. The separator body includes a first sealing perimeter for providing sealing engagement with a first portion of a collection container while allowing a sample to pass through the through-hole into the collection container, and a second sealing perimeter for providing sealing engagement with a second portion of the collection container while maintaining a barrier for separation between the first and second phases.

The separation assembly may include a closure adapted for sealing engagement with the open end of the collection container, in which the mechanical separator is releasably engaged with a portion of the closure.

In accordance with another embodiment of the present invention, a separation assembly for enabling separation of a fluid sample into first and second phases includes a collection container having an open end, a closed end, and a sidewall extending therebetween defining an interior. The collection container further defines a longitudinal axis between the open end and the closed end. The separation assembly further includes a closure adapted for sealing engagement with the open end of the collection container, and a post engaged with the closure and adapted for positioning within the interior of the collection container. The post includes a post through-hole aligned along the longitudinal axis of the collection container. The separation assembly also includes a mechanical separator releasably engaged with the post. The mechanical separator includes a separator body having a through-hole defined therein along a through-axis, with the through-hole adapted for allowing fluid to pass therethrough. The separator body includes a float, having a first density, and a ballast, having a second density greater than the first density. A portion of the float is connected to a portion of the ballast, and a portion of the post is received within the through-hole of the separator forming a fluid path through the post and the mechanical separator in an initial first position.

The separator body may further include an initial engagement band circumferentially disposed about a portion of the separator body. The initial engagement band and the float may be formed of the same material, and the initial engagement band may bisect at least a portion of the ballast. Optionally, the separator body is adapted to transition from a first initial position in which a portion of the post is disposed within the through-hole and the separator body is oriented in an open position for allowing fluid to pass therethrough, to a second sealing position in which the separator body is disengaged from the post and the through-hole is oriented in a closed position for preventing fluid from being received therethrough, upon applied rotational force. Transition of the separator body from the open position to the closed position may include an axial movement of the separator body to disengage from the post, and a rotational movement of the separator body from an initial first position to a second sealing position.

In accordance with yet another embodiment of the present invention, a separation assembly for enabling separation of a fluid sample into first and second phases includes a collection container having an open end, a closed end, and a sidewall extending therebetween defining an interior. The collection container further defines a longitudinal axis between the open end and the closed end. The separation assembly further includes a closure adapted for sealing engagement with the open end of the collection container. The closure includes a receiving end for positioning within the open end of the collection container, with the receiving end defining an interior cavity and including an undercut protrusion extending into the interior cavity. The separation assembly further includes a mechanical separator releasably engaged with the closure. The mechanical separator includes a separator body having a through-hole defined therein along a through-axis, with the through-hole adapted for allowing fluid to pass therethrough. The separator body includes a float, having a first density, and a ballast, having a second density greater than the first density, with a portion of the float connected to a portion of the ballast. The undercut protrusion of the closure may be disposed within the through-hole of the separator, and at least a portion of the separator body may be disposed within the interior cavity of the closure in an initial first position.

In accordance with yet another embodiment of the present invention, a collection container includes a first region having an open top end and a first sidewall defining a first interior and a first exterior. The collection container also includes a second region having a closed bottom end and a second sidewall defining a second interior and a second exterior. The first region and the second region may be aligned along a longitudinal axis such that the first interior and the second interior are provided in fluid communication. A diameter of the first interior may be greater than a diameter of the second interior, and at least one fluid flute may extend between the first region and the second region to allow passage of fluid therethrough from the first region to the second region.

In certain configurations, the first exterior has a 16 mm profile and the second exterior has a 13 mm profile. The first interior may be dimensioned to accommodate a mechanical separator therein, and the second interior may be dimensioned to at least partially restrain a portion of the mechanical separator from passing therein absent applied rotational force.

In accordance with yet another embodiment of the present invention, a separation assembly for enabling separation of a fluid sample into first and second phases includes a collection container having a first region having an open top end and a first sidewall defining a first interior and a first exterior, and a second region having a closed bottom end and a second sidewall defining a second interior and a second exterior. The first region and the second region may be aligned along a longitudinal axis such that the first interior and the second interior are provided in fluid communication, with a diameter of the first interior being greater than a diameter of the second interior. The separation assembly further includes at least one fluid flute extending between the first region and the second region to allow passage of fluid therethrough from the first region to the second region. The separation assembly may also include a mechanical separator having a float, having a first density, and a ballast, having a second density greater than the first density, with a portion of the float connected to a portion of the ballast. At least a portion of the mechanical separator is prevented from entering the second region in an initial first position, and the mechanical separator is transitioned into the second region upon application of rotational force to a second sealing position.

The mechanical separator may include a separator body having a through-hole defined therein and adapted for allowing fluid to pass therethrough.

In accordance with still a further embodiment of the present invention, a separation assembly for enabling separation of a fluid sample into first and second phases includes a collection container having a first end, a second end, and a sidewall extending therebetween defining an interior. The separation assembly further includes a closure adapted for sealing engagement with the open end of the collection container. The separation assembly also includes a mechanical separator releasably restrained by at least one of the closure and the sidewall of the collection container in an initial first position. The mechanical separator includes a separator body having a through-hole defined therein along a through-axis, with the through-hole adapted for allowing fluid to pass therethrough. The separator body includes a float, having a first density, and a ballast, having a second density greater than the first density, with a portion of the float connected to a portion of the ballast. The separation assembly further includes a carrier releasably engaged with a portion of the mechanical separator in the initial position such that, upon application of rotational force, the separator body transitions from an initial position in which fluid may pass through the through-hole, to a sealing position in which the mechanical separator prevents passage of fluid therethrough or therearound. Also upon application of rotational force, the carrier disengages from the mechanical separator.

In still a further embodiment of the present invention, a separation assembly includes a separation assembly including a collection container having a first end, a second end, and a sidewall extending therebetween defining an interior. The separation assembly also includes a mechanical separator including a float and a ballast and capable of movement from a first position to a sealing position. In the sealing position, a sealing perimeter is established between at least a portion of the interior and the separator, the sealing perimeter having a varying position about a portion of the interior, with the varying position defining an average sealing height. The mechanical separator also has a maximum height and a minimum height within the collection container, such that the average sealing height is less than the maximum height minus the minimum height.

The assembly of the present invention is advantageous over existing separation products that utilize separation gel. In particular, the assembly of the present invention will not interfere with analytes, whereas many gels interact with bodily fluids and/or analytes present within a collection container. The assembly of the present invention is also advantageous over existing mechanical separators in that the separator does not require piercing of the separator body to introduce a specimen into the collection container thereby minimizing pre-launch and sample pooling under the closure. The structure of the present mechanical separator also minimizes the loss of trapped fluid phases, such as serum and plasma within the separator body. Additionally, the assembly of the present invention does not require complicated extrusion techniques during fabrication, and may optimally employ two-shot molding techniques.

Further details and advantages of the invention will become clear from the following detailed description when read in conjunction with the accompanying drawings.

### BRIEF DESCRIPTION OF THE DRAWINGS

**FIG. 1** is a partial cross-sectional side view of a conventional mechanical separator.
**FIG. 2** is a perspective view of a mechanical separator assembly having a float defining a through-hole and a ballast in accordance with an embodiment of the present invention.
**FIG. 3** is an alternative perspective view of the mechanical separator assembly of FIG. 2.
**FIG. 4** is a top view of the mechanical separator of **FIG. 2****.**
**FIG. 5** is a side view of the mechanical separator of **FIG. 2****.**
**FIG. 6** is a cross-sectional view of the mechanical separator of **FIG. 2** taken along line **A-A** of **FIG. 5****.**
**FIG. 7** is a front view of the mechanical separator of **FIG. 2****.**
**FIG. 8** is a cross-sectional view of the mechanical separator of **FIG. 2** taken along line **B-B** of **FIG. 7****.**
**FIG. 9** is a top view of an alternative mechanical separator having a float defining a through-hole and a ballast, with first and second extended tabs forming a substantially convex upper float surface in accordance with an embodiment of the present invention.
**FIG. 10** is a side view of the mechanical separator of **FIG. 9****.**
**FIG. 11** is a cross-sectional view of the mechanical separator of **FIG. 9** taken along line **C-C** of **FIG. 10****.**
**FIG. 12** is a front view of the mechanical separator of **FIG. 9****.**
**FIG. 13** is a cross-sectional view of the mechanical separator of **FIG. 9** taken along line **D-D** of **FIG. 12****.**
**FIG. 14** is a perspective view of an alternative mechanical separator having a float defining an elliptical through-hole and a ballast in accordance with an embodiment of the present invention.
**FIG. 15** is an alternative perspective view of the mechanical separator of **FIG. 14****.**
**FIG. 16** is a top view of the mechanical separator of **FIG. 15****.**
**FIG. 17** is a side view of the mechanical separator of **FIG. 15****.**
**FIG. 18** is a cross-sectional view of the mechanical separator of **FIG. 15** taken along line **E-E** of **FIG. 17****.**
**FIG. 19** is a front view of the mechanical separator of **FIG. 15****.**
**FIG. 20** is a cross-sectional view of the mechanical separator of **FIG. 15** taken along line **F-F** of **FIG. 19****.**
**FIG. 20A** is a perspective view of a mechanical separator having a spheroid shaped body and a reduced separation between the first extended tab and the second extended tab in accordance with an embodiment of the present invention.
**FIG. 21** is a cross-sectional view of an alternative mechanical separator having an elliptical interior taken along a similar cross-sectional line as that shown in **FIG. 18****.**
**FIG. 22** is a partial perspective view of the mechanical separator having an elliptical interior as shown in **FIG. 21****.**
**FIG. 23** is a cross-sectional view of an alternative mechanical separator having an elliptical through-hole taken along a similar cross-sectional line as that shown in **FIG. 18****.**
**FIG. 24** is a partial perspective view of the mechanical separator having an elliptical through-hole as shown in **FIG. 23****.**
**FIG. 25** is a cross-sectional view of an alternative mechanical separator having a substantially round interior and side-cuts taken along a similar cross-sectional line as that shown in **FIG. 18****.**
**FIG. 26** is a partial perspective view of the mechanical separator having a substantially round interior and side-cuts as shown in **FIG. 25****.**
**FIG. 27** is a partial cross-sectional side view of a mechanical separator of the present invention affixed to a closure in accordance with an embodiment of the present invention.
**FIG. 28** is a partial cross-sectional side view of a mechanical separator disposed within a collection container in an initial position for allowing fluid to pass through the through-hole in accordance with an embodiment of the present invention.
**FIG. 29** is a partial cross-sectional side view of a mechanical separator disposed within a collection container as shown in **FIG. 28** in a sealing position for establishing a barrier between lighter and denser phases within a collection container after application of rotational force in accordance with an embodiment of the present invention.
**FIG. 30** is a perspective view of a mechanical separator in accordance with an embodiment of the present invention having a seal line for engagement with a collection container in an initial position.
**FIG. 31** is a perspective view of the mechanical separator of **FIG. 30** having a seal line for engagement with a collection container in a sealing position.
**FIG. 31A** is a perspective view of a mechanical separator having a partially scalloped surface in accordance with an embodiment of the present invention.
**FIG. 31B** is a front view of the mechanical separator of **FIG. 31A****.**
**FIG. 31C** is a perspective view of a mechanical separator in accordance with an embodiment of the present invention.
**FIG. 31D** is a top view of the mechanical separator of **FIG. 31C****.**
**FIG. 31E** is a front view of the mechanical separator of **FIG. 31C****.**
**FIG. 31F** is a cross-sectional view of the mechanical separator of **FIG. 31C** taken along line **31F-31F** of **FIG. 31E****.**
**FIG. 31G** is a side view of the mechanical separator of **FIG. 31C****.**
**FIG. 31H** is a cross-sectional view of the mechanical separator of **FIG. 31C** taken along line **31H-31H** of **FIG. 31G****.**
**FIG. 31I** is a bottom view of the mechanical separator of **FIG. 31C****.**
**FIG. 32** is a perspective view of a mechanical separator having an initial engagement band in accordance with an embodiment of the present invention.
**FIG. 33** is an alternative perspective view of a mechanical separator having an initial engagement band as shown in **FIG. 32****.**
**FIG. 34** is a side view of the mechanical separator having an initial engagement band as shown in **FIG. 33****.**
**FIG. 35** is a partial cross-sectional side view of the mechanical separator having an initial engagement band of **FIG. 33** engaged with a portion of the sidewall of a collection container and closure in accordance with an embodiment of the present invention.
**FIG. 35A** is a perspective view of a mechanical separator having an extended tab band in accordance with an embodiment of the present invention.
**FIG. 35B** is a left side view of the mechanical separator of **FIG. 35A****.**
**FIG. 35C** is a front view of the mechanical separator of **FIG. 35A****.**

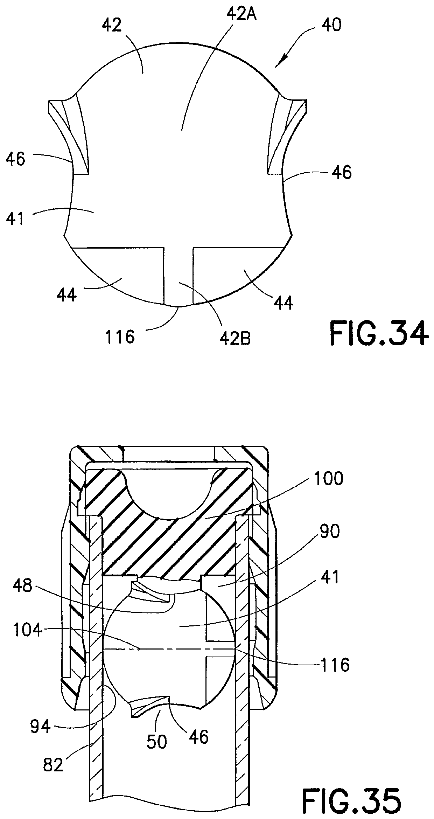

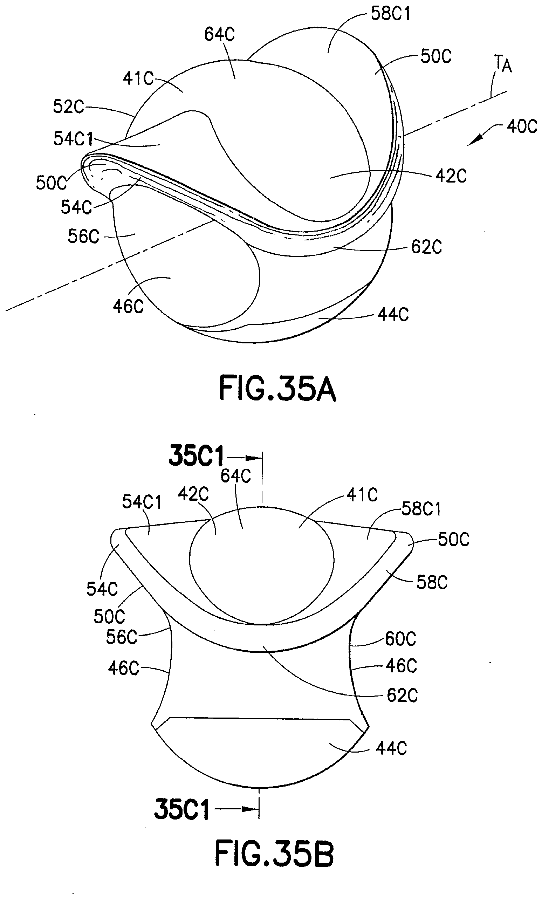

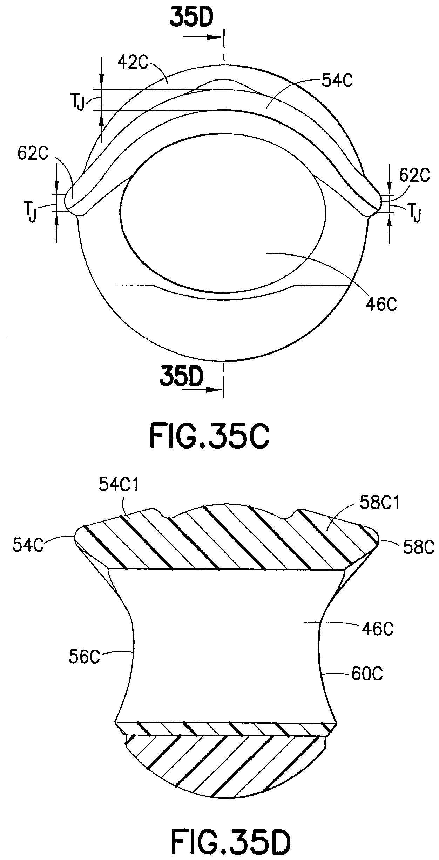

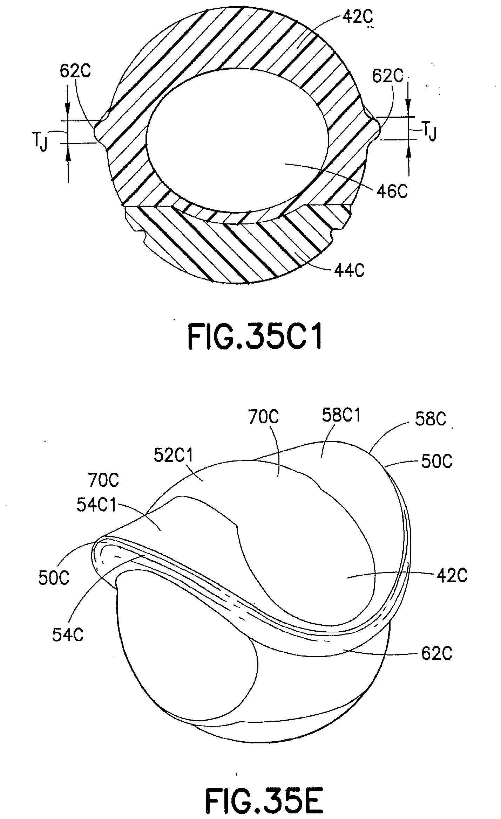

**FIG. 35C1** is a cross-sectional view of the mechanical separator of **FIG. 35A** taken along line **35C1-35C1** of **FIG. 35B****.**
**FIG. 35D** is a cross-sectional view of the mechanical separator of **FIG. 35A** taken along line **35D-35D** of **FIG. 35C****.**
**FIG. 35E** is a perspective view of a mechanical separator having an alternative extended tab band in accordance with an embodiment of the present invention.

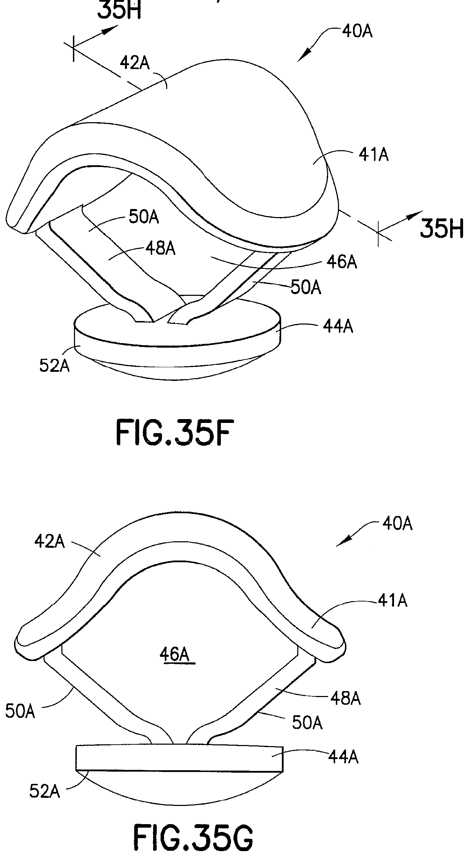

**FIG. 35F** is a perspective view of a mechanical separator having a joining structure in accordance with an embodiment of the present invention.
**FIG. 35G** is a front view of the mechanical separator of **FIG. 35F****.**

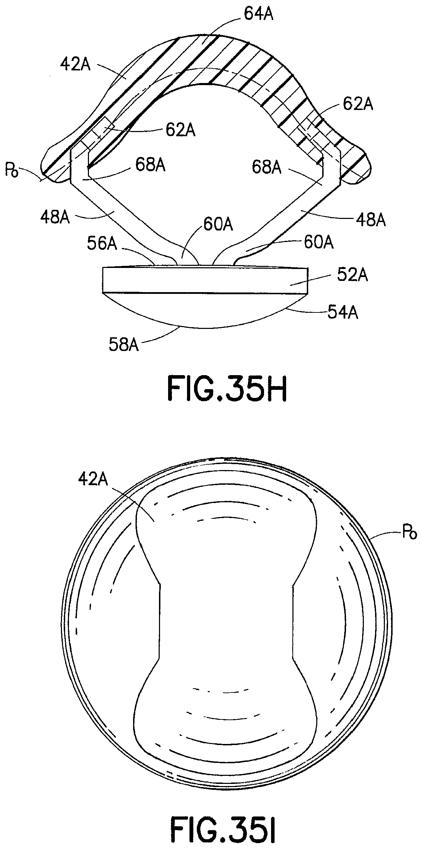

**FIG. 35H** is a cross-sectional view of the mechanical separator of **FIG. 35G** taken along line **35H-35H** of **FIG. 35F****.**
**FIG. 35I** is a top view of the mechanical separator of **FIG. 35F****.**

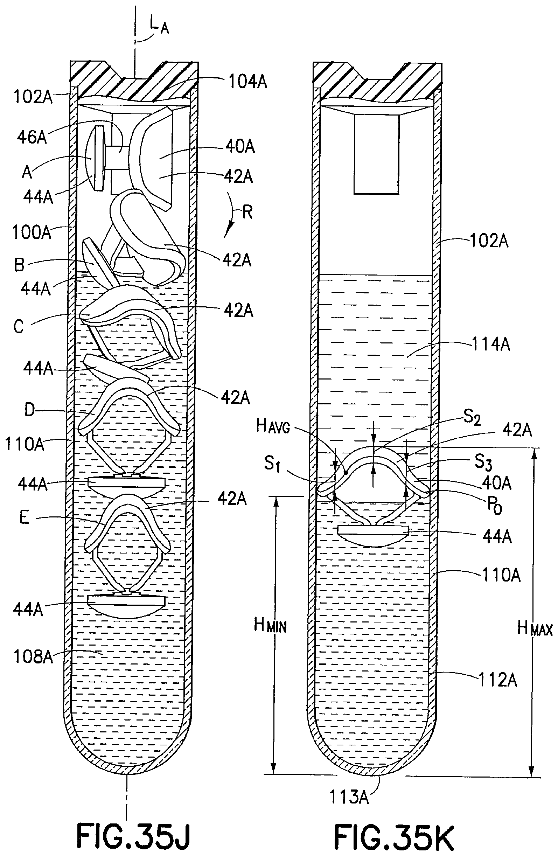

**FIG. 35J** is a schematic front view of the mechanical separator of **FIG. 35F** disposed within a collection container in various states of descent within the collection container in accordance with an embodiment of the present invention.
**FIG. 35K** is a schematic front view of the mechanical separator of **FIG. 35J** in a sealing position in accordance with an embodiment of the present invention.

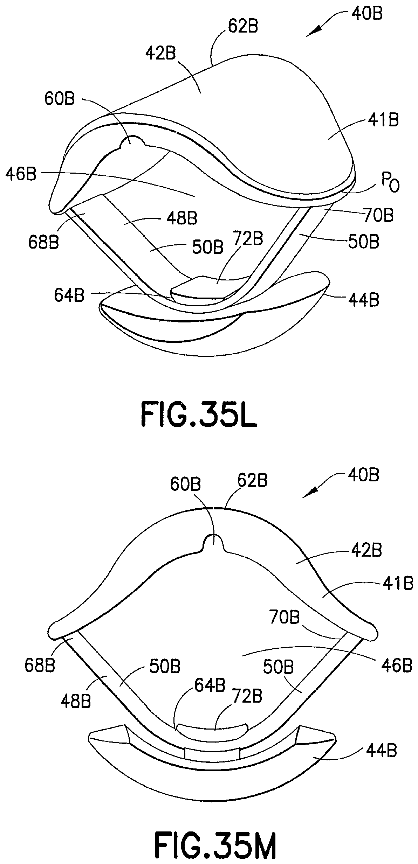

**FIG. 35L** is a perspective view of a mechanical separator having an alternative joining structure in accordance with an embodiment of the present invention.
**FIG. 35M** is a front view of the mechanical separator of **FIG. 35L****.**

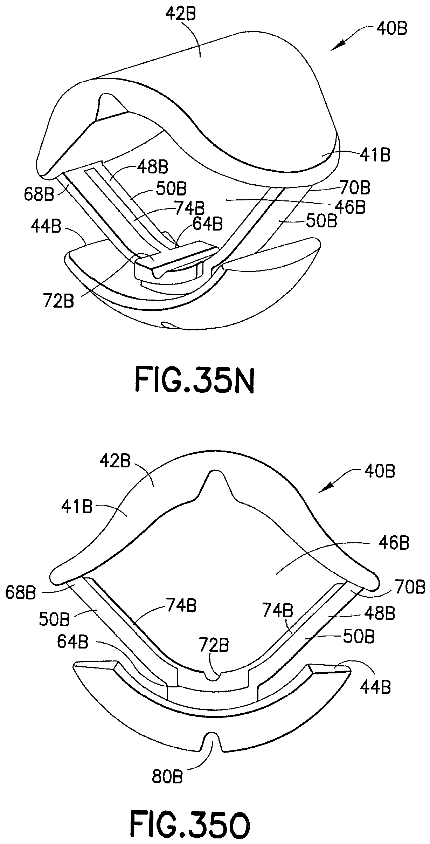

**FIG. 35N** is a perspective view of a mechanical separator having an alternative joining structure in accordance with an embodiment of the present invention.
**FIG. 35O** is a front view of the mechanical separator of **FIG. 35N****.**

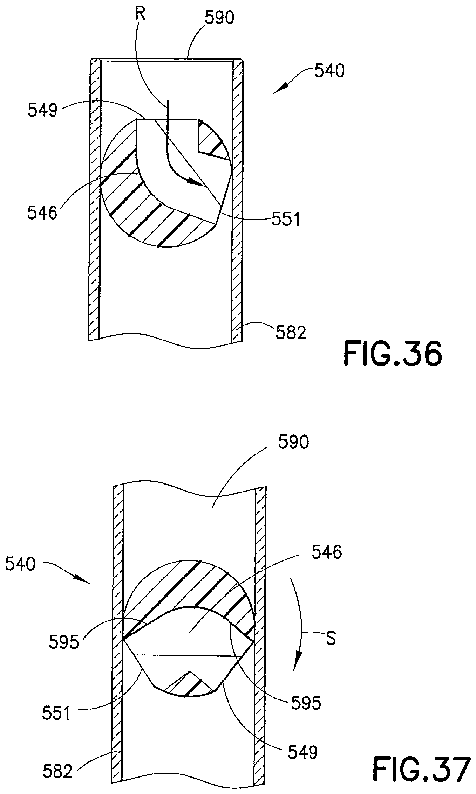

**FIG. 36** is a partial cross-sectional side view of a mechanical separator having a circuitous though-hole in an initial position in accordance with an embodiment of the present invention.
**FIG. 37** is a partial cross-sectional side view of the mechanical separator of **FIG. 36** having a circuitous though-hole in a sealing position in accordance with an embodiment of the present invention.

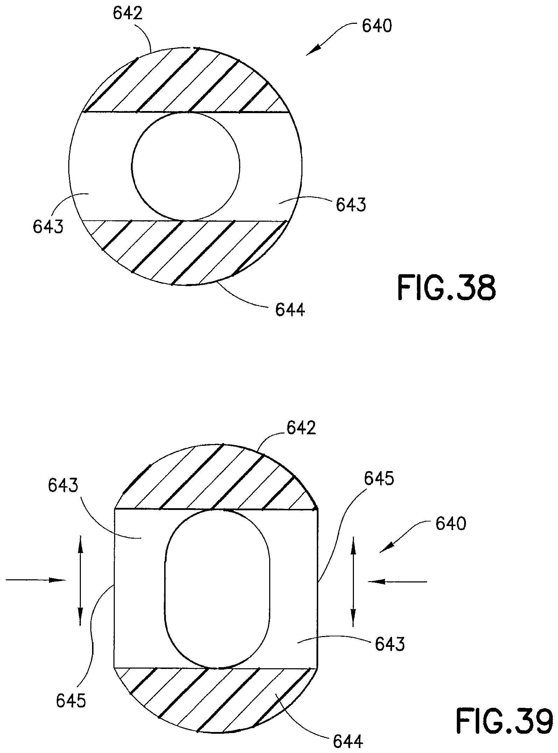

**FIG. 38** is a representational cross-section of a mechanical separator having a float and a ballast separated by a thermoplastic elastomer section defining a through-hole in an initial resting position in accordance with yet another embodiment of the present invention.
**FIG. 39** is a representational cross-section of the mechanical separator of **FIG. 38** having a float and a ballast separated by a thermoplastic elastomer section defining a through-hole in an activated position during application of rotational force.

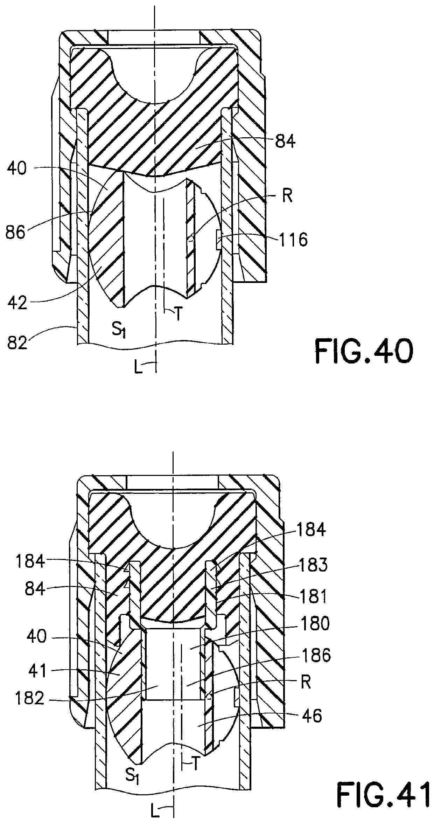

**FIG. 40** is a cross-sectional side view of a separation assembly having a mechanical separator engaged with a portion of a collection container having a closure engaged therewith in accordance with an embodiment of the present invention.
**FIG. 41** is a cross-sectional side view of an alternative separation assembly having a mechanical separator engaged with a post which is engaged with an undercut of closure in accordance with an embodiment of the present invention.

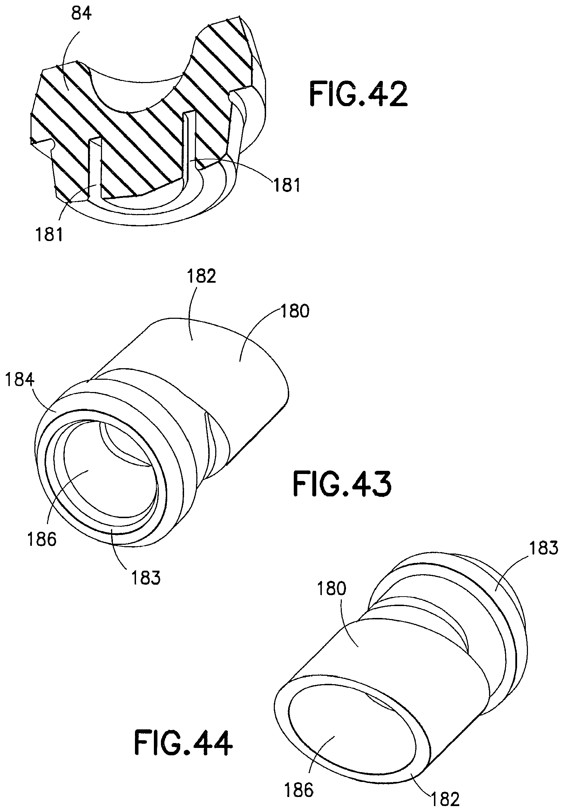

**FIG. 42** is a partial cross-sectional perspective of the closure of **FIG. 41****.**
**FIG. 43** is a perspective front view of the post of **FIG. 41****.**
**FIG. 44** is a perspective rear view of the post of **FIG. 41****.**

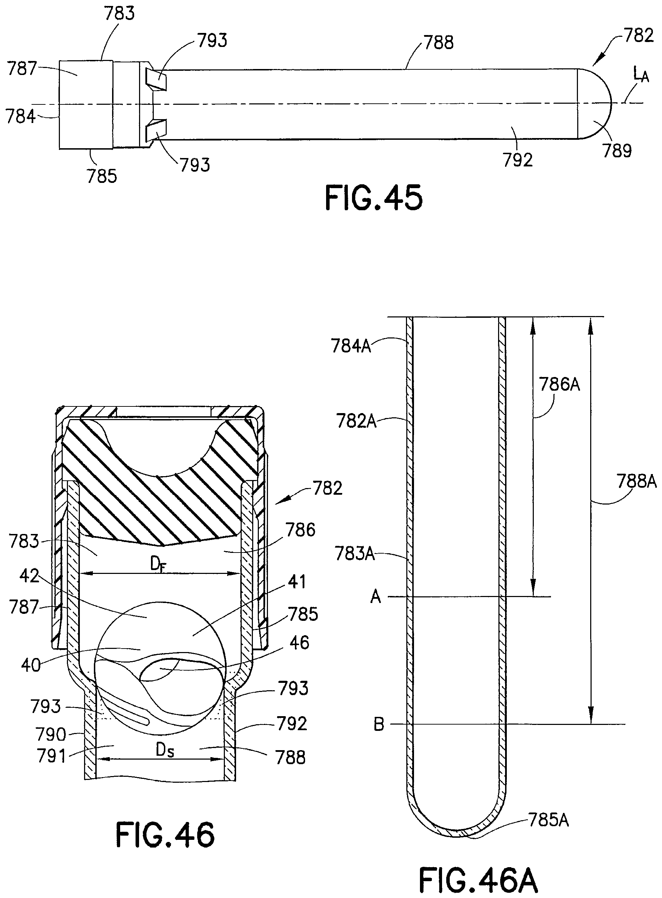

**FIG. 45** is a side view of a collection container having a first region, a second region, and a plurality of fluid flutes in accordance with an embodiment of the present invention.
**FIG. 46** is a cross-sectional partial side view of a separation assembly having a mechanical separator disposed within the collection container of **FIG. 45** in accordance with an embodiment of the present invention.
**FIG. 46A** is a cross-sectional side view of an alternative collection container for use with a mechanical separator in accordance with an embodiment of the present invention.

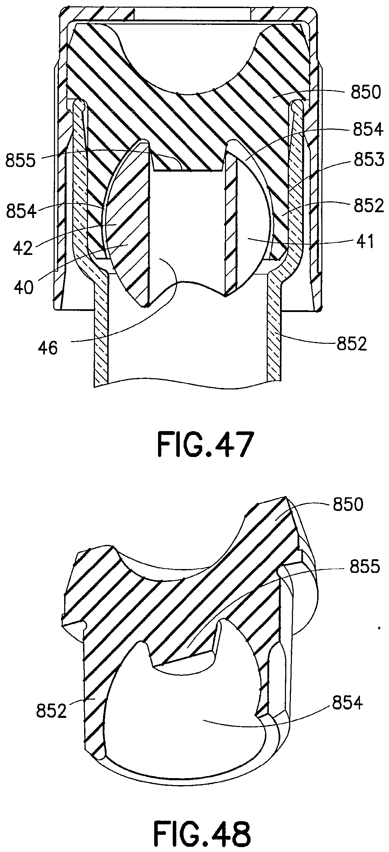

**FIG. 47** is a cross-sectional side view of an alternative separation assembly having a mechanical separator engaged within a portion of a closure in accordance with an embodiment of the present invention.
**FIG. 48** is a partial cross-sectional perspective of the closure of **FIG. 47****.**

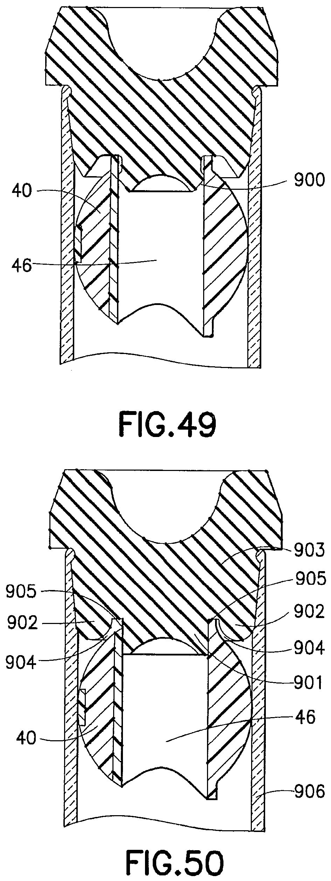

**FIG. 49** is a cross-sectional side view of a separation assembly having a mechanical separator engaged with a closure having an engagement boss in accordance with an embodiment of the present invention.
**FIG. 50** is a cross-sectional side view of an alternative separation assembly having a mechanical separator engaged with a closure having an alternative engagement boss in accordance with an embodiment of the present invention.

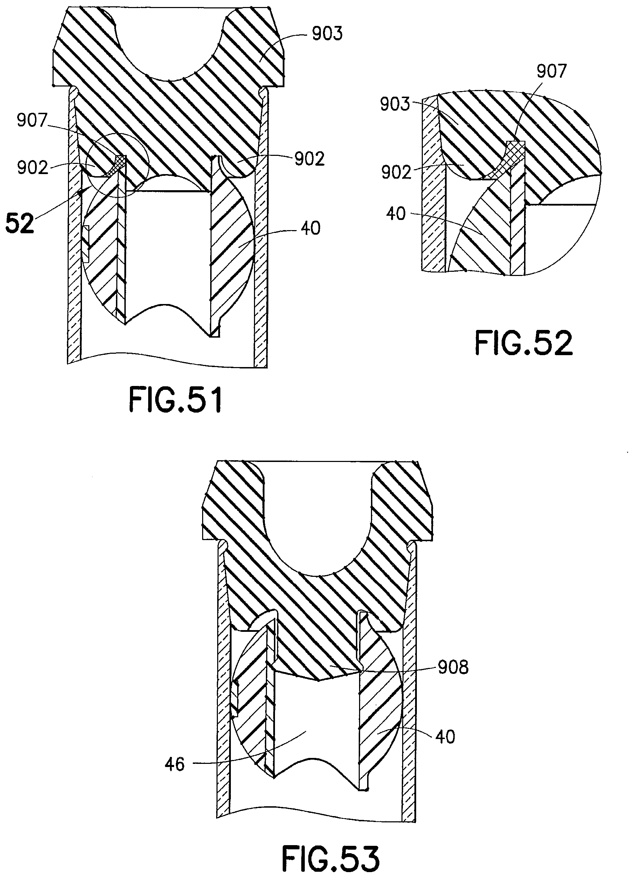

**FIG. 51** is a cross-sectional side view of the separation assembly of **FIG. 50** having a sealant disposed between a portion of the mechanical separator and a portion of the closure in accordance with an embodiment of the present invention.
**FIG. 52** is a close-up sectional view of the sealant shown in **FIG. 51****.**
**FIG. 53** is a cross-sectional side view of an alternative separation assembly having a mechanical separator engaged with a closure having an alternative engagement boss in accordance with an embodiment of the present invention.

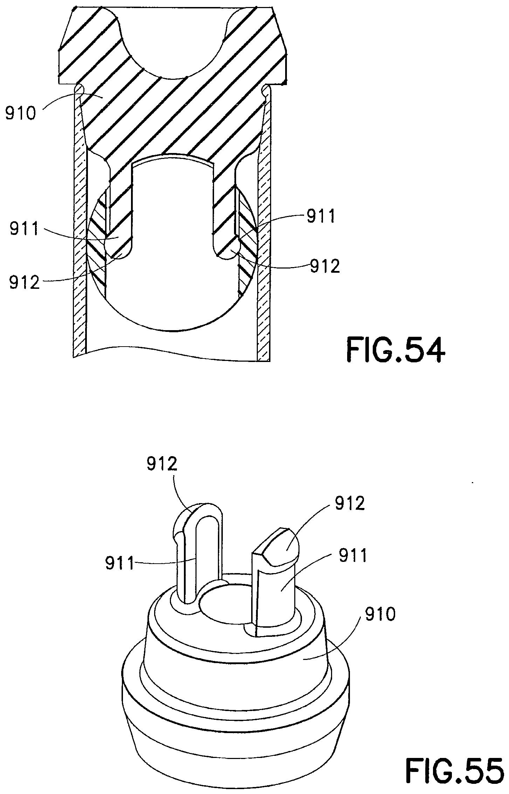

**FIG. 54** is a cross-sectional side view of an alternative separation assembly having a mechanical separator engaged with a closure having an alternative engagement boss in accordance with an embodiment of the present invention.
**FIG. 55** is a perspective view of the closure of **FIG. 54** having an engagement boss including a plurality of depending feet.

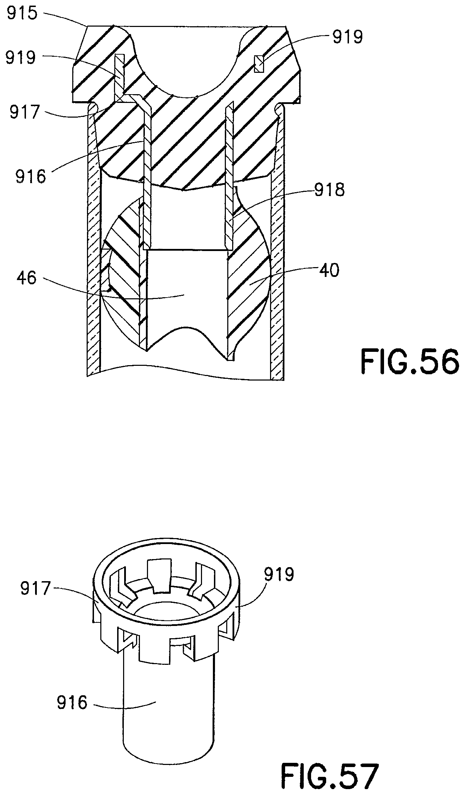

**FIG. 56** is a cross-sectional side view of an alternative separation assembly having a mechanical separator engaged with a molding insert in accordance with an embodiment of the present invention.
**FIG. 57** is a perspective view of the molding insert of **FIG. 56****.**

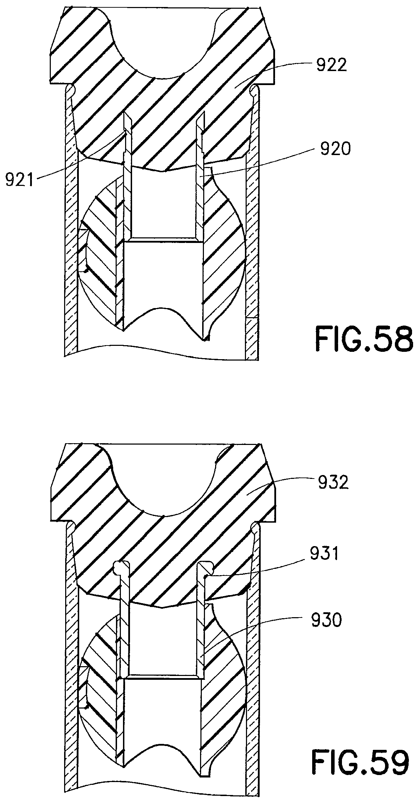

**FIG. 58** is a cross-sectional side view of an alternative separation assembly having a mechanical separator engaged with a molding insert in accordance with an embodiment of the present invention.
**FIG. 59** is a cross-sectional side view of an alternative separation assembly having a mechanical separator engaged with a molding insert in accordance with an embodiment of the present invention.

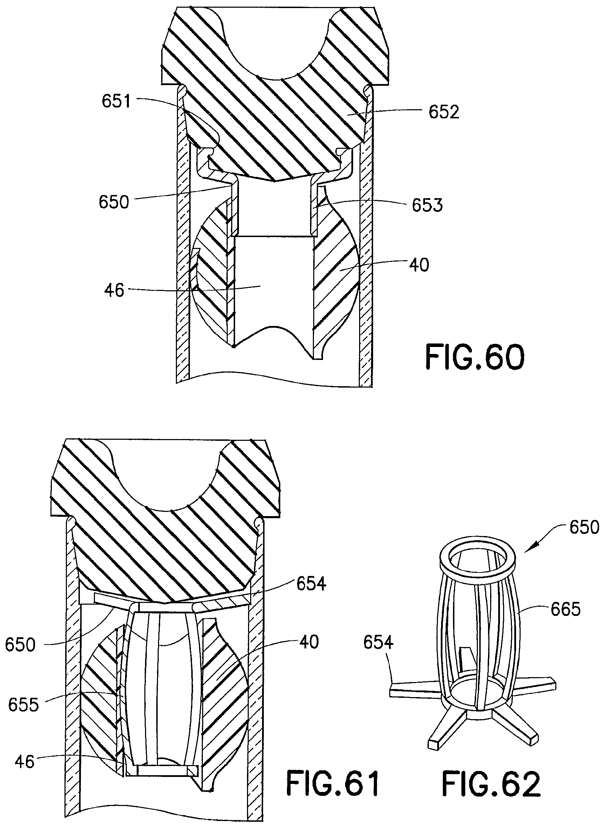

**FIG. 60** is a cross-sectional side view of an alternative separation assembly having a mechanical separator engaged with a carrier engaged with a portion of the closure in accordance with an embodiment of the present invention.
**FIG. 61** is a cross-sectional side view of an alternative separation assembly having a mechanical separator engaged with an alternative carrier engaged with a portion of the closure in accordance with an embodiment of the present invention.
**FIG. 62** is a perspective view of the carrier of **FIG. 61****.**

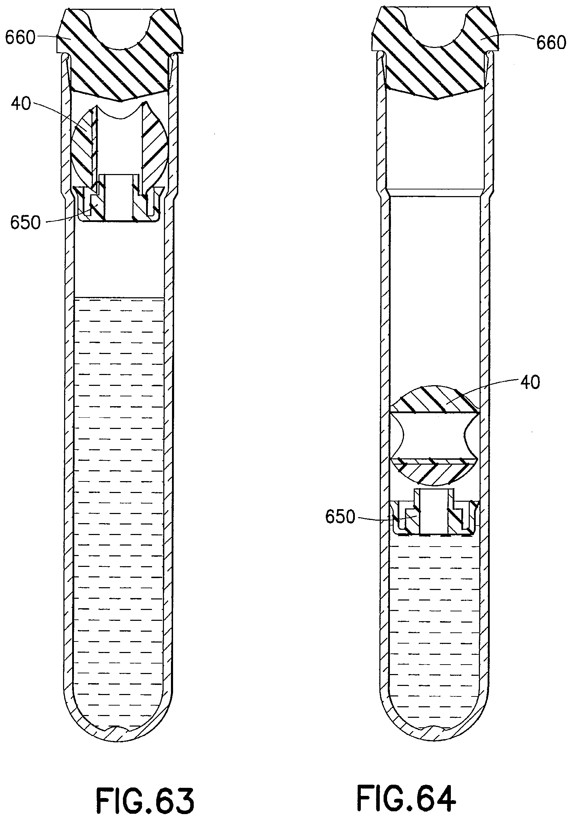

**FIG. 63** is a cross-sectional side view of a separation assembly having a mechanical separator engaged with a carrier in an initial position in accordance with an embodiment of the present invention.
**FIG. 64** is a cross-sectional side view of the separation assembly of **FIG. 63** having a mechanical separator in a sealing position disengaged from the carrier after application of rotational force in accordance with an embodiment of the present invention.

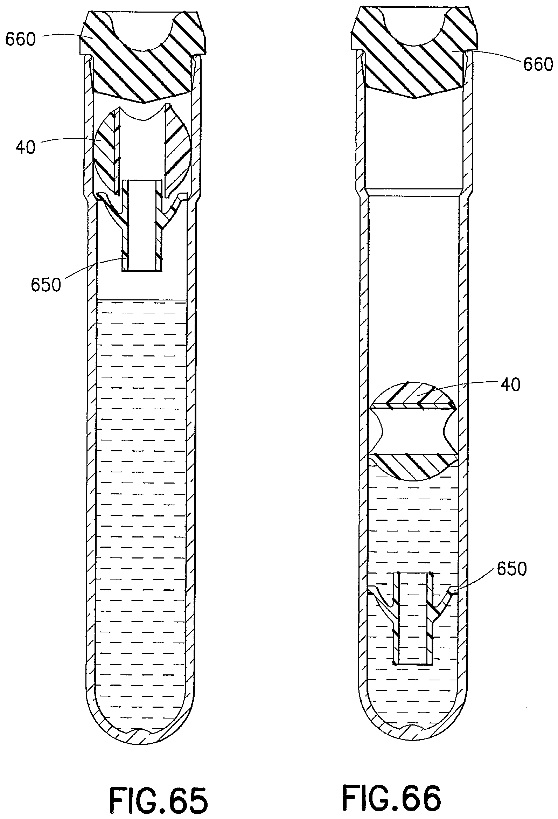

**FIG. 65** is a cross-sectional side view of an alternative separation assembly having a mechanical separator engaged with an alternative carrier in an initial position in accordance with an embodiment of the present invention.
**FIG. 66** is a cross-sectional side view of the separation assembly of **FIG. 65** having a mechanical separator in a sealing position disengaged from the carrier after application of rotational force in accordance with an embodiment of the present invention.

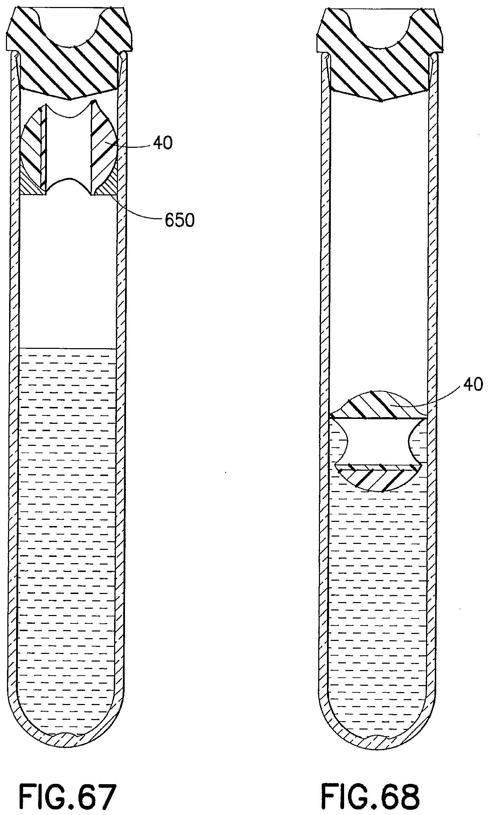

**FIG. 67** is a cross-sectional side view of an alternative separation assembly having a mechanical separator engaged with a dissolvable carrier in an initial position in accordance with an embodiment of the present invention.
**FIG. 68** is a cross-sectional side view of the separation assembly of **FIG. 67** having a mechanical separator in a sealing position illustrating the carrier in the fully dissolved state after application of rotational force in accordance with an embodiment of the present invention.

### DESCRIPTION OF THE PREFERRED EMBODIMENTS

For purposes of the description hereinafter, the words "upper", "lower", "right", "left", "vertical", "horizontal", "top", "bottom", "lateral", "longitudinal", and like spatial terms, if used, shall relate to the described embodiments as oriented in the drawing figures. However, it is to be understood that many alternative variations and embodiments may be assumed except where expressly specified to the contrary. It is also to be understood that the specific devices and embodiments illustrated in the accompanying drawings and described herein are simply exemplary embodiments of the invention.

The mechanical separator of the present invention is intended for use with a collection container for providing separation of a sample into higher and lower density phase components, as will be discussed herein. For example, the present mechanical separator can be used to provide a separation of serum or plasma from whole blood through the use of differential buoyancy to cause a sealing area to contract when submerged in a specimen exposed to elevated gravitational forces through applied rotational force or centrifugation. In one embodiment, the elevated gravitational forces can be provided at a rate of at least 2,000 revolutions/minute, such as at least 3,400 revolutions/minute.

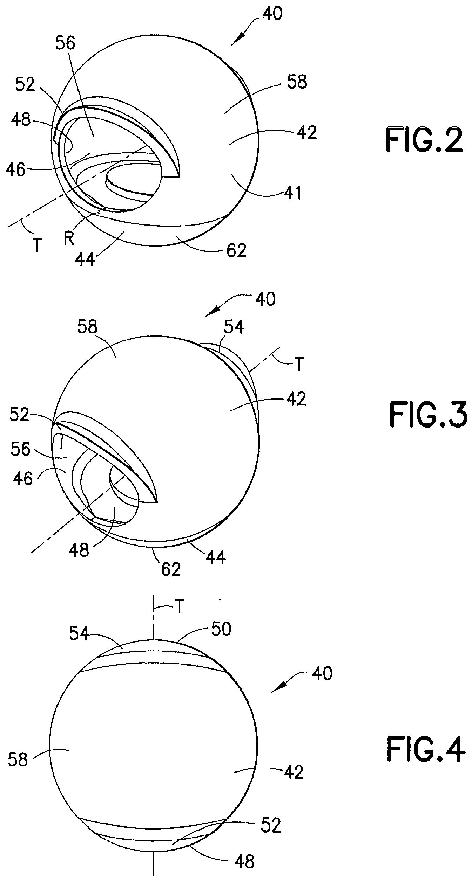

Referring to **FIGS. 2-8****,** the mechanical separator **40** of the present invention includes a separator body **41** including a float **42** and a ballast **44** connected to the float **42.** In one embodiment, the float **42** has a first density and the ballast **44** has a second density, with the second density being greater than the first density. In another embodiment, the float **42** has a first buoyancy and the ballast **44** has a second buoyancy, with the first buoyancy being greater than the second buoyancy. In one embodiment, it is desirable that the float **42** of the mechanical separator **40** be made from a material having a density that is lighter than the liquid or specimen intended to be separated into two phases. For example, if it is desired to separate human blood into serum and plasma, then it is desirable that the float **42** have a density of no more than about 1.020 g/cc. In one configuration, the float **42** of the mechanical separator **40** may be extruded and/or molded of a resiliently deformable and self-sealable material, such as a thermoplastic elastomer (TPE). In yet another embodiment, the float **42** may be extruded and/or molded of a resiliently deformable material that exhibits good sealing characteristics when contact is established with a collection container, as will be discussed herein. Maintenance of the float density within the specified tolerances is more easily obtained by using a standard material that does not require compounding with, for example, glass micro-spheres in order to reduce the material density.

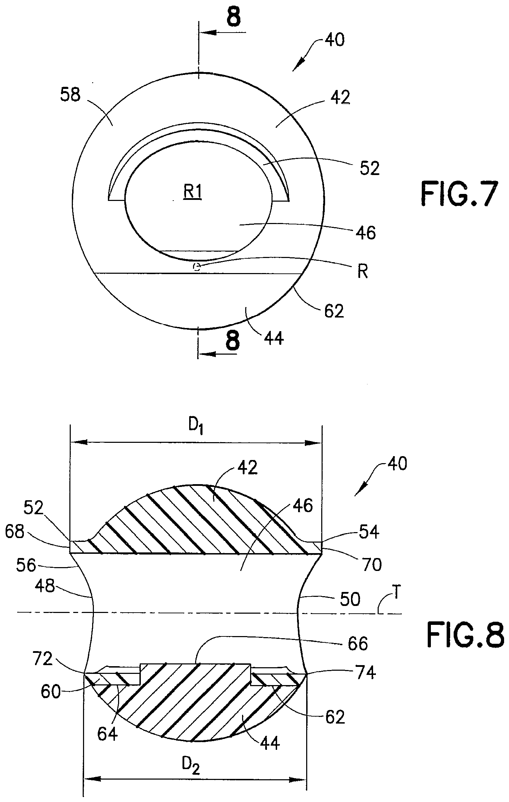

The mechanical separator **40** also includes a through-hole **46** defined therein, such as along a through-axis **T** of the separator body **41.** As shown in **FIGS. 3****,** **5****,** and **8****,** the through-hole **46** may extend through the entire separator body **41** and includes a first opening **48** and a second opening **50** aligned along the through-axis **T.** In one configuration, the through-hole **46** bisects or substantially bisects the volumetric center of the separator body **41.** In one embodiment, the through-hole **46** is disposed entirely within the float **42.** In a further embodiment, the float **42** may further include a first extended tab **52** adjacent the first opening **48** of the through-hole **46,** and a second extended tab **54** adjacent the second opening **50** of the through-hole **46.** The first extended tab **52** and/or the second extended tab **54** may be co-formed with the float **42**, forming a portion of the float **42** itself. In another configuration, the first extended tab **52** and/or the second extended tab **54** may be separately formed and subsequently joined with the float **42.** The first extended tab **52** and the second extended tab **54** may be provided above, such as substantially above, the through-axis T of the separator body **41.** The first extended tab **52** and the second extended tab **54** may also be provided about, such as substantially about, a portion of the through-hole **46,** such as in an outwardly-extending arcuate shape about an upper portion **56** of the through-hole **46.** The first extended tab **52** and the second extended tab **54** may extend outwardly from the float **42** in a direction parallel or substantially parallel to the through axis **T** of the separator body **41,** such that the first extended tab **52** and the second extended tab **54** may have the same shape and curvature or substantially the same shape and curvature. In yet another embodiment, as shown in **FIG. 8****,** the first extended tab **52** includes a first outermost edge **68** at the upper outermost portion of a first side of the through-hole **46,** and the second extended tab **54** includes a second outermost edge **70** at the corresponding upper outermost portion of a second side of the through-hole **46.** In one configuration, the first outermost edge **68** extends outwardly a distance that is greater than the lower outermost portion **72** of the first side of the through-hole **46.** The second outermost edge **70** also extends outwardly a distance that is greater than the corresponding lower outermost portion **74** of the second side of the through-hole **46.** Accordingly, the diameter **D₁** of the separator body **41** taken about the first extended tab **52** and the second extended tab **54** about an upper portion of the through-hole **46** is slightly greater than the diameter **D₂** of the separator body **41** taken about the lower portion of the through-hole **46** defined by the lower outermost portions **72, 74.**

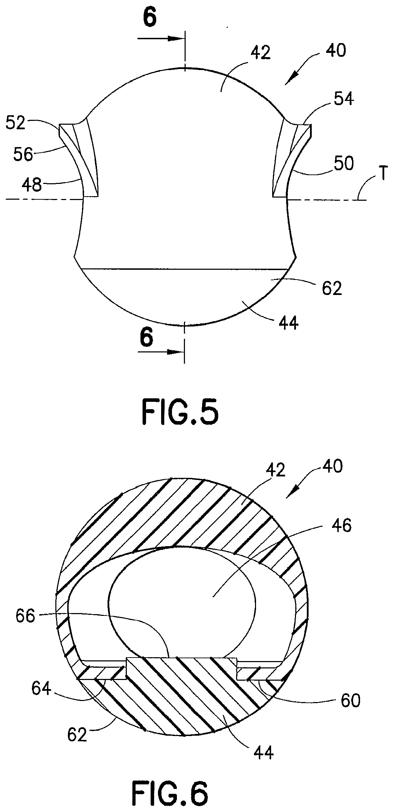

In one embodiment, the float **42** has an exterior surface **58** that is generally arcuate in shape, such as at least partially rounded or substantially rounded, and a joining surface **60,** shown in **FIGS. 6** and **8****,** adapted for engagement with a portion of the ballast **44.** The ballast **44** also includes an exterior surface **62** that is also generally arcuate in shape, such as at least partially rounded or substantially rounded, and a contact surface **64,** also shown in **FIGS. 6** and **8****,** that is adapted for joining with the joining surface **60** of the float **42.** In one embodiment, when taken together, the exterior surface **58** of the float **42** and the exterior surface **62** of the ballast **44** form a generally round exterior, such as a spheroid shape. It is understood herein that the term "spheroid shape" may include other configurations, in addition to a perfect sphere, that are aspects of the invention which may provide slightly nonuniform diameters taken through the mid-point. For example, different planes taken through the float **42** and ballast **44** which bisect the midpoint of the mechanical separator 40 may have varying diameter and still give rise to a generally rounded or ball-like mechanical separator **40** having a spheroid shape. In one embodiment, the float **42** and the ballast **44** may be separately formed and subsequently assembled. In another embodiment, the float **42** and the ballast **44** may be co-formed, such as co-extruded and/or co-molded, such as by a two-shot or multi-shot molding process such that both components are integrally linked together to form a complete separator body **41.** In another configuration, this integral linkage between the float **42** and the ballast **44** may be created by a material bond between the two components, by a mechanical interlock, or by a combination of a material bond and a mechanical interlock. In addition, the float **42** and the ballast **44** may be linked together by a separate post-molding operation, such as adhesive, heat-staking, and/or ultrasonic welding. As shown in **FIGS. 6** and **8****,** the ballast **44** may include an attachment protrusion **66** which assists in the engagement of the ballast **44** and the float **42.**

In one embodiment, it is desirable that the ballast **44** of the mechanical separator **40** be made from a material having a higher density than the liquid intended to be separated into two phases. For example, if it is desired to separate human blood into serum and plasma, then it is desirable that the ballast **44** have a density of at least 1.029 g/cc. In one embodiment, the ballast **44** can be formed from mineral filled polypropylene. It is anticipated herein that both the float **42** and the ballast **44** could be formed of various other materials with sufficient biocompatibility, density stability, additive compatibility, and neutrality to analyte interactions, adsorption, and leachability.

Due to the differential densities of the float **42** and the ballast **44,** the mechanical separator **40** includes a center of mass **R** that is offset from the center of volume **R1** of the separator body **41.** Specifically, the volume of the separator body **41** accounted for by the float **42** may be significantly greater than the volume of the separator body **41** accounted for by the ballast **44.** Accordingly, in certain embodiments, the center of mass **R** of the separator body **41** may be offset from the through-hole **46.**

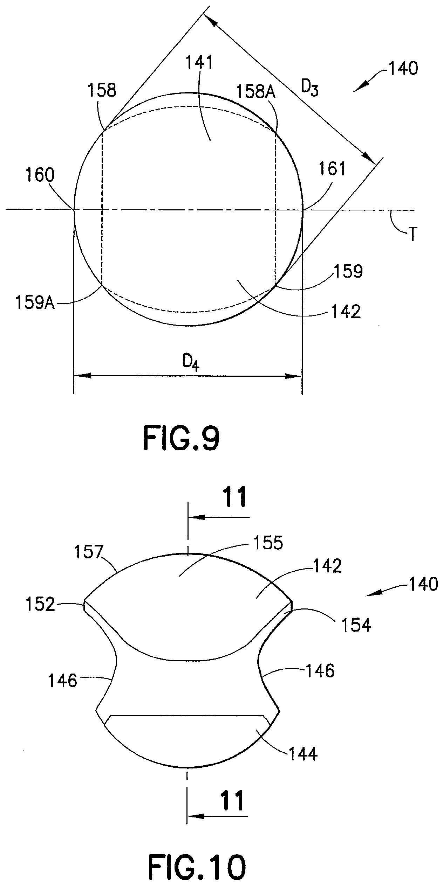

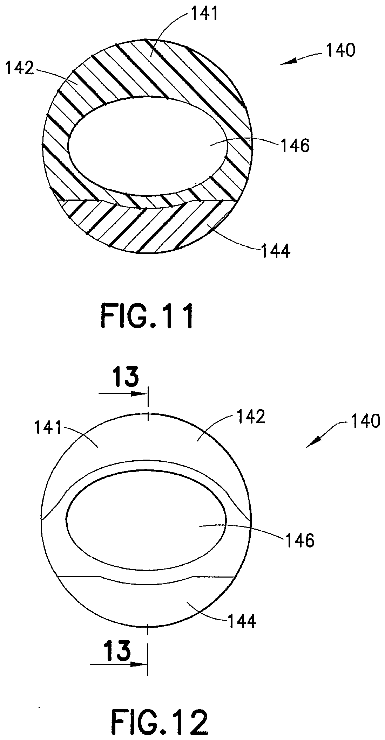

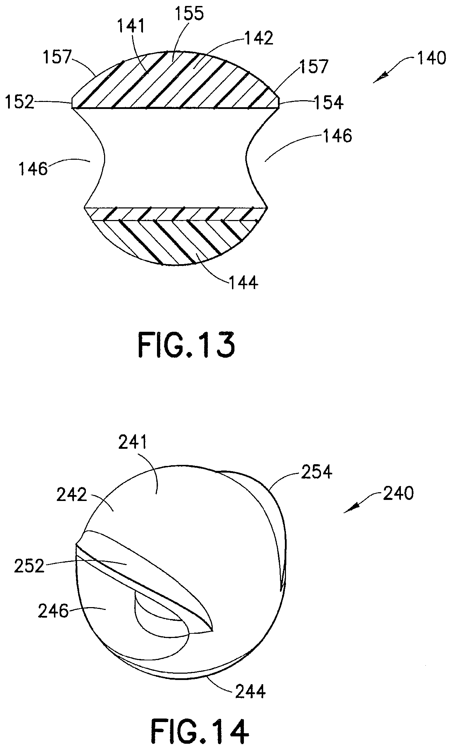

In accordance with another embodiment of the present invention, as shown in **FIGS. 9-13****,** the mechanical separator **140** includes a separator body **141** having a float **142** and a ballast **144** with a through-hole **146** defined within the float **142,** as discussed above. In this configuration, shown specifically in **FIGS. 10** and **13****, the** first extended tab **152** and the second extended tab **154,** taken with an upper portion **155** of the float **142,** form a substantially convex upper float surface **157.** As shown in **FIG. 9****,** the profile of the separator body **141** is slightly off-spherical such that a diameter **D₃** of the separator body extending between diagonally off-set endpoints **158, 159** of the through-hole **146** extending along the through-axis **T,** is slightly larger than a diameter **D₄** of the separator body extending between outermost opposing endpoints **160, 161** tangent to the perimeter of the separator body **141** and perpendicular to the through-hole **146.** Accordingly, the endpoints (diagonally off-set endpoints **158, 159,** and second diagonally off-set endpoints **158A, 159A**) may each include a thickened area of material, such as TPE.

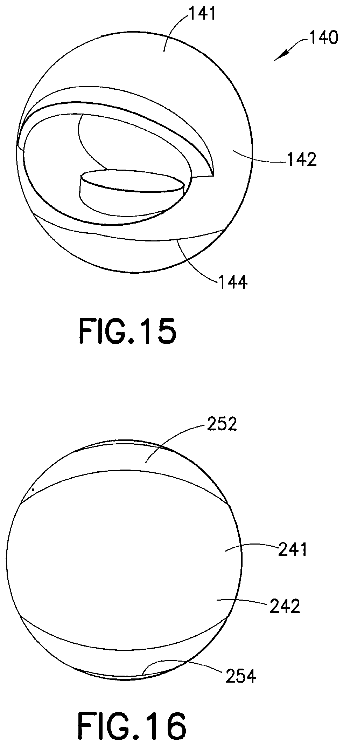

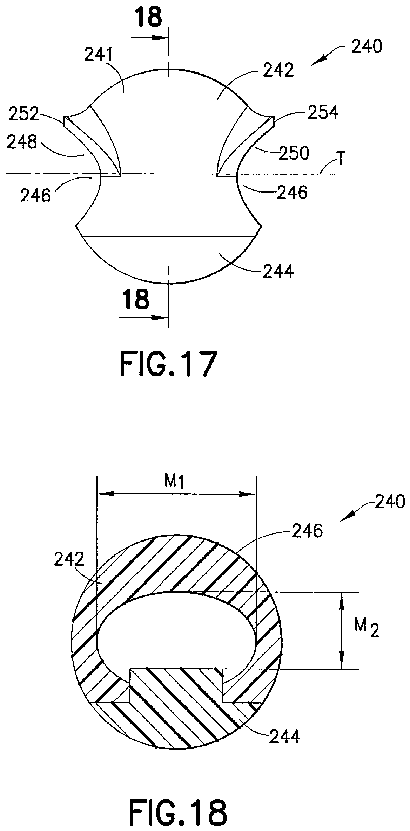

In accordance with another embodiment, as shown in **FIGS. 14-20****,** the mechanical separator **240** includes a separator body **241** having a float **242** and a ballast **244** with a through-hole **246** defined within the float **242,** as discussed above. In this configuration, the through-hole **246** may have a substantially elliptical cross-section, as specifically shown in **FIGS. 18-19****.** In one embodiment, the major axis **M₁** of the ellipse, shown in **FIG. 18****,** is oriented perpendicular to the through-axis **T,** shown in **FIG. 17****.** By extending the major axis **M₁** of the ellipse perpendicular to the through-axis **T,** the float **242** may be adapted for increased elongation in the direction of the minor axis **M₂** (shown in **FIG. 18****)** of the ellipse upon application of rotational force, as will be discussed herein.

In this configuration, the curvature of the first extended tab **252** and the curvature of the second extended tab **254** are elongated to substantially mimic at least a portion of the elliptical first opening **248** and second opening **250** of the through-axis **T,** respectively. In another embodiment, the first extended tab **252** is at least partially curved in shape, such as having a convex shape, and is provided adjacent the upper portion of the first opening **248** of the through-hole **246.** The second extended tab **254** may also be at least partially curved in shape, such as having a convex shape, and may be provided adjacent the upper portion of the second opening **250** of the through-hole **246.**

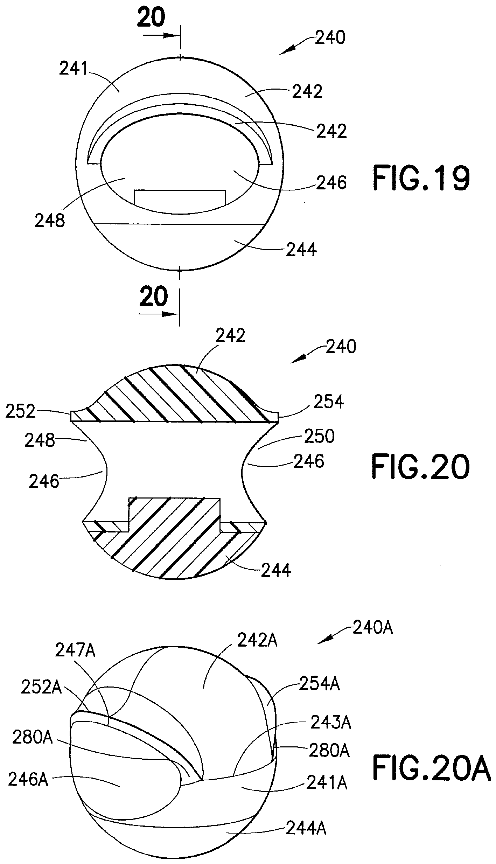

As shown in **FIG. 20A****,** the mechanical separator **240A** includes a separator body **241A** having a float **242A** and a ballast **244A** with a through-hole **246A** defined within the float **242A,** as discussed above. In this configuration, the first extended tab **252A** and the second extended tab **254A** may have an elliptical profile that is substantially coincident to the diameter **243A** of the separator body **241A** at the edges of the through-hole **246A,** and slightly offset from the diameter **243A** at the apex **247A** of the first and second extended tabs **252A, 254A.** In this configuration, the first extended tab **252A** and the second extended tab **254A** may include enlarged fillets **280A** positioned at the edges of the first and second extended tabs **252A, 254A** adjacent the through-hole **246A** to assist in the formation of a barrier against a portion of the tube wall in the sealing position, as described herein. The enlarged fillets **280A** may function to facilitate the shedding of cells around the mechanical separator during application of applied rotational force, as described herein. The enlarged fillets **280A** may also include a region of the first and second extended tabs **252A, 254A** having an increased thickness and/or diameter, such as a widened taper adjacent the ends of the first and second extended tabs **252A, 254A** and extending along at least a portion of the through-hole **246A.**

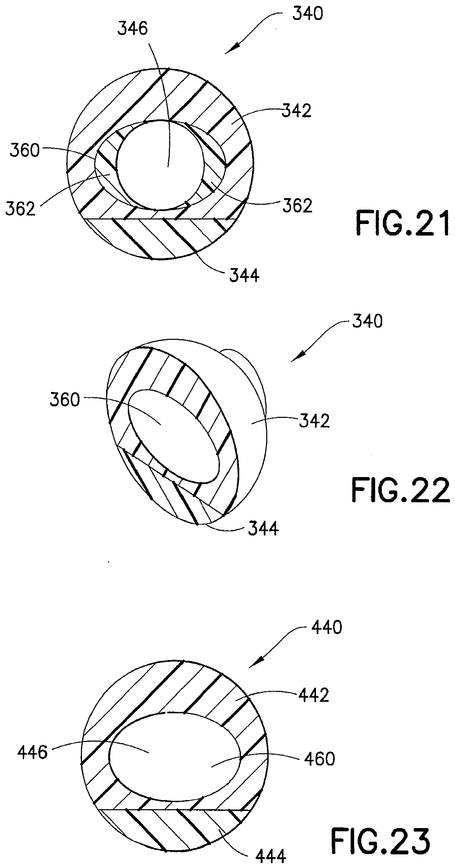

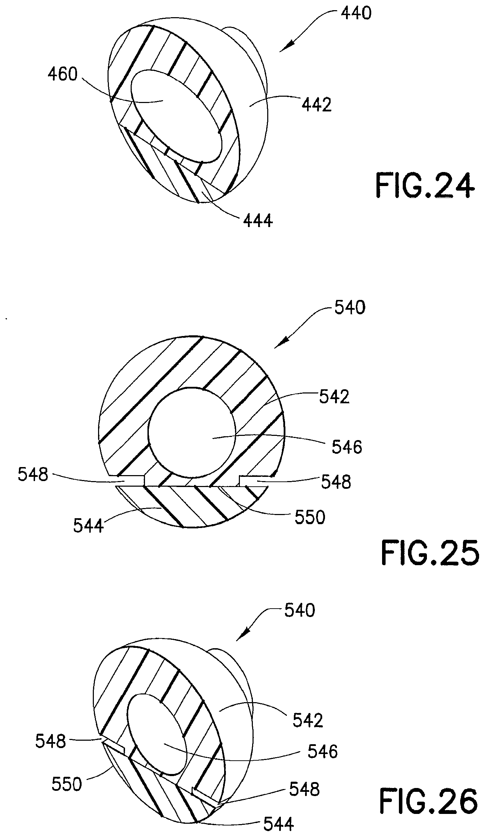

As shown in **FIGS. 21-22****,** a mechanical separator **340** of the present invention includes a float **342** and a ballast **344,** and may include an elliptical interior **360** defining a substantially cylindrical through-hole **346.** In this configuration, the elliptical interior **360** may include a filler material **362** dimensioned to fill the elliptical interior **360** leaving a substantially cylindrical though-hole **346.** In one embodiment, the filler material **362** may be a TPE material or other sufficiently flexible material. Alternatively, as shown in **FIGS. 23-****24,** a mechanical separator **440** of the present invention, including a float **442** and a ballast **444,** may include an elliptical interior **460** defining an elliptical through-hole **446.** In yet another configuration, a mechanical separator **540** of the present invention, including a float **542** and a ballast **544,** may include a through-hole **546** having a circular cross-section and a cylindrical shape. Optionally, the float **542** may also include a slit **548** or plurality of slits **548,** such as adjacent an interface **550** with the ballast **544.** The inclusion of a slit **548** or a plurality of slits **548** defined within the float **542** may provide for increased elongation of the float **542** upon application of rotational force, as will be discussed herein.

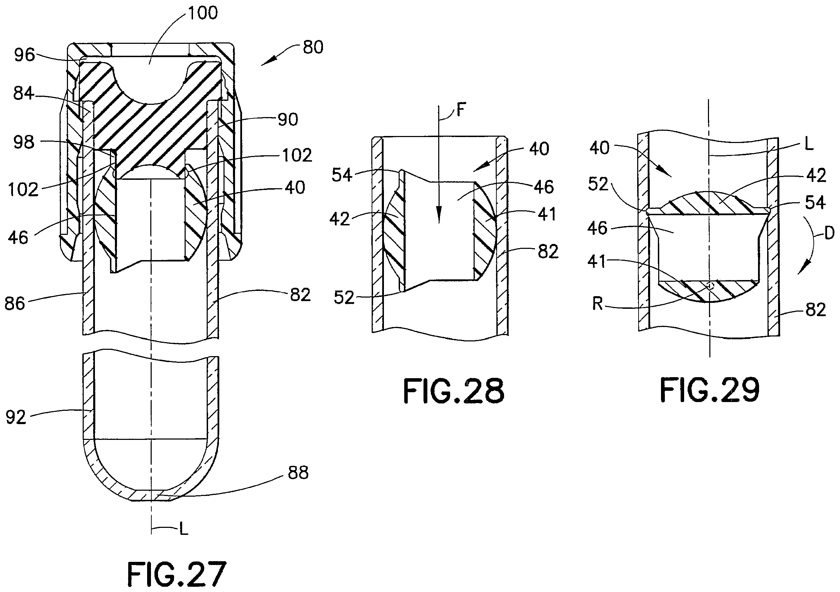

As shown in **FIG. 27****,** the mechanical separator **40** of the present invention may be provided as a portion of a separation assembly **80** for separating a fluid sample into first and second phases within a collection container **82** having a closure **84.** Specifically, the collection container **82** may be a sample collection tube, such as a proteomics, molecular diagnostics, chemistry sample tube, blood, or other bodily fluid collection tube, coagulation sample tube, hematology sample tube, and the like. Desirably, collection container **82** is an evacuated blood collection tube. In one embodiment, the collection container **82** may contain additional additives as required for particular testing procedures, such as protease inhibitors, clotting agents, and the like. Such additives may be in particle or liquid form and may be sprayed onto the cylindrical sidewall **86** of the collection container **82** or located at the bottom of the collection container **82.** The collection container **82** includes a closed bottom end **88,** an open top end **90,** and a cylindrical sidewall **92** extending therebetween. The cylindrical sidewall **92** includes an inner surface **94** with an inside diameter extending substantially uniformly from the open top end **90** to a location substantially adjacent the closed bottom end **88** along the longitudinal axis **L** of the collection container **82.**

The collection container **82** may be made of one or more than one of the following representative materials: polypropylene, polyethylene terephthalate (PET), glass, or combinations thereof. The collection container **82** can include a single wall or multiple wall configurations. Additionally, the collection container **82** may be constructed in any practical size for obtaining an appropriate biological sample. For example, the collection container **82** may be of a size similar to conventional large volume tubes, small volume tubes, or microtainer tubes, as is known in the art. In one particular embodiment, the collection container **82** may be a standard 13 ml evacuated blood collection tube, as is also known in the art.

The open top end **90** is structured to at least partially receive the closure **84** therein to form a liquid impermeable seal. The closure **84** includes a top end **96** and a bottom end **98** structured to be at least partially received within the collection container **82.** Portions of the closure **84** adjacent the top end **90** define a maximum outer diameter which exceeds the inside diameter of the collection container **82.** In one embodiment, the closure **84** includes a pierceable resealable septum **100** penetrable by a needle cannula (not shown). Portions of the closure **84** extending downwardly from the bottom end **98** may taper from a minor diameter which is approximately equal to, or slightly less than, the inside diameter of the collection container **82** to a major diameter that is greater than the inside diameter of the collection container **82** at the top end **96.** Thus, the bottom end **98** of the closure **84** may be urged into a portion of the collection container **82** adjacent the open top end **90.** The inherent resiliency of closure **84** can insure a sealing engagement with the inner surface **94** of the cylindrical sidewall **86** of the collection container **82.** In one embodiment, the closure **84** can be formed of a unitarily molded elastomeric material, having any suitable size and dimensions to provide sealing engagement with the collection container **82.** Optionally, the closure **84** may be at least partially surrounded by a shield, such as a Hemogard® Shield commercially available from Becton, Dickinson and Company.

As shown in **FIG. 27****,** the mechanical separator **40** of the present invention may be oriented within the collection container **82** in an initial position in which the through-hole **46** of the mechanical separator **40** is aligned with the open top end **90** of the collection container **82.** In the initial position, the through-hole **46** is adapted for allowing fluid to pass therethrough, such as from a needle cannula (not shown) which has pierced the pierceable septum **100** of the closure **84** and is provided in fluid communication with the interior of the collection container **82.** The mechanical separator **40** may also be releasably engaged with a portion of the closure **84** such that the separator body **41** may transition from the initial position, as shown in **FIGS. 27-28****,** to a sealing position, as shown in **FIG. 29****.** In the initial position, the through-hole **46** is oriented in an open position for allowing fluid to pass therethrough in the direction indicated in **FIG. 28** by flow arrow **F.** Referring to **FIG. 27****,** the initial open position of the through-hole **46** is substantially aligned with the longitudinal axis **L** of the collection container **82.** Referring to **FIG. 29****,** upon application of rotational force, such as during centrifuge, the mechanical separator **40** deforms sufficiently to disengage from engagement with the closure **84** and rotate in the direction shown by directional arrow **D** of **FIG. 29** to the sealing position in which the through-hole **46** is in a substantially closed position. In the substantially closed position, the float **42** including the first extended tab **52** and the second extended tab **54** form a sealing engagement with the inner surface **94** of the collection container **82** substantially preventing fluid from being received through the through-hole **46** or around the separator body **41.**

In one configuration, the through-hole **46 is** substantially aligned with the open top end 90 of the collection container **82** along at least a portion of the longitudinal axis **L** in the open position, and the through-hole **46** is substantially aligned perpendicular to the longitudinal axis in the closed position. It is noted that transition of the through-hole **46** from the open position to the closed position coincides with the rotation of the mechanical separator **40** from a first initial position to a second closed position. In another configuration, the mechanical separator **40** is engaged with a portion of the closure **84** in the first initial position, and the mechanical separator **40** is engaged with a portion of the sidewall **86** of the collection container **82** in the second sealing position. Referring again to **FIG. 27****,** the closure **84** may include an engagement boss **102** for engagement with the mechanical separator **40.** In one configuration, the engagement boss **102** is disposed within a portion of the through-hole **46** when the separator body **41** is in the first initial position for forming a fluid seal between a portion of the separator body **41** and the closure **84.**

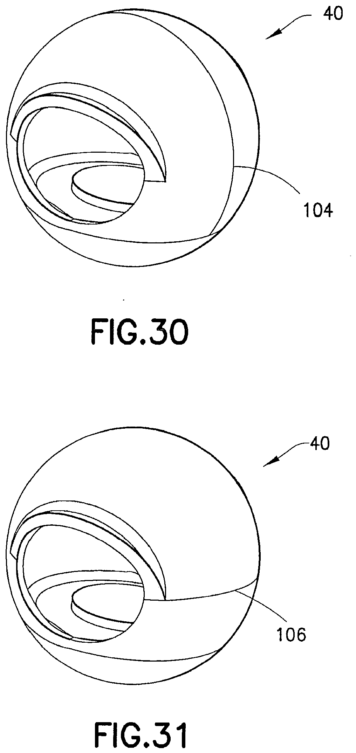

In the initial position, the mechanical separator **40** may be attached to the closure **84** be means of a mechanical snap created by an undercut in the through-hole **46** which controls the release load of the mechanical separator **40.** When the mechanical separator **40** is attached to the closure **84,** it forms a seal with the sidewall **86** of the collection container **82** along a first sealing perimeter **104** as shown in **FIG. 30****.** During specimen draw into the collection container **82,** the first sealing perimeter **104** prevents the accumulation of blood between the mechanical separator **40** and the closure **84.** This reduces the formation of clots and/or fibrin strands which may disrupt function of the mechanical separator **40.** Upon application of rotational force and transition of the mechanical separator **40** as shown in **FIG. 29****,** the mechanical separator **40** experiences a rotational moment while still attached to the closure **84** and, after release from the closure **84,** rotates approximately 90° to become oriented with the ballast **44** facing the bottom end **88** of the collection container **82.**

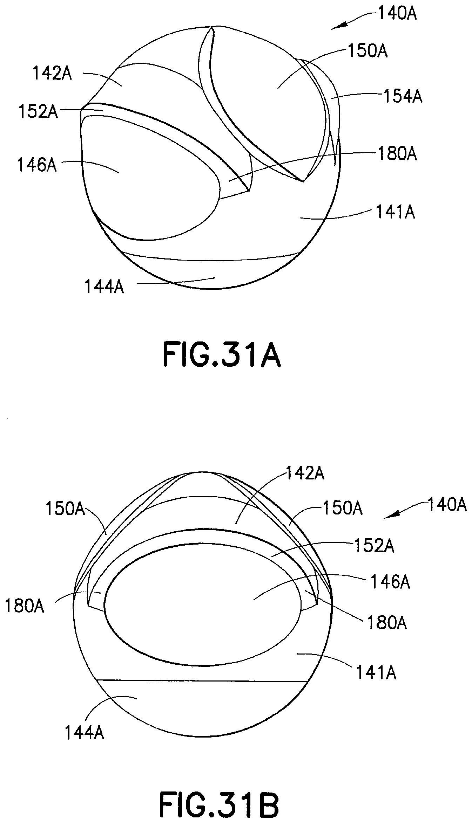

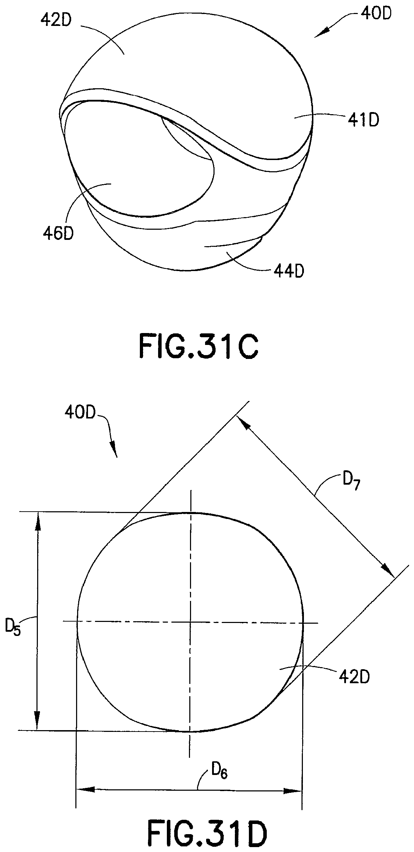

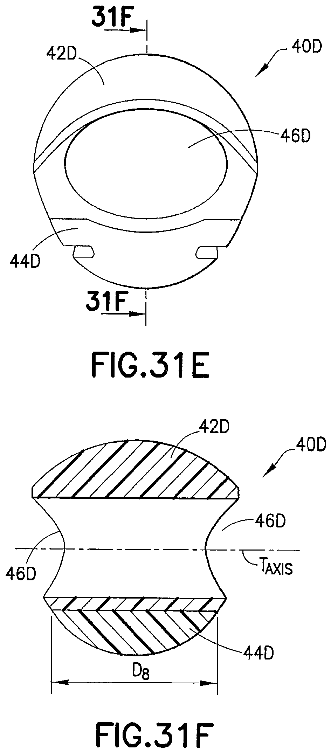

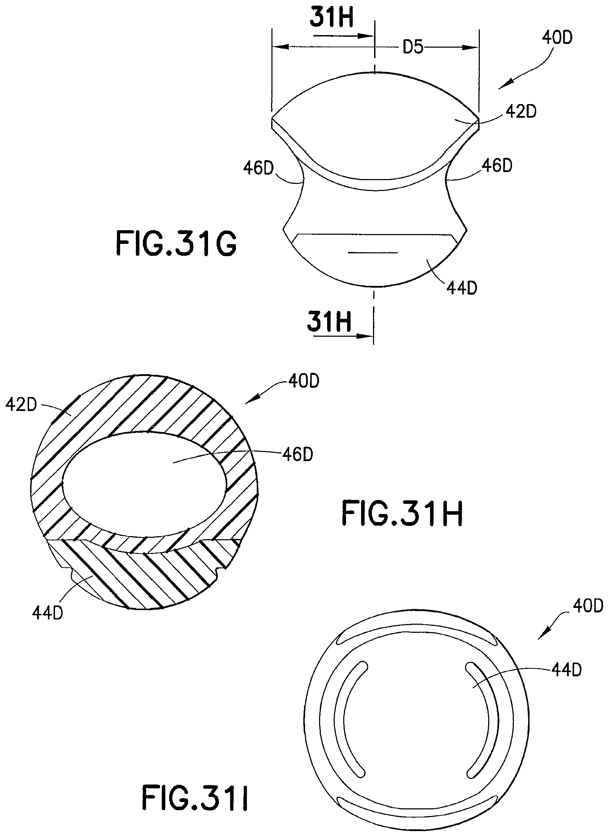

Once the mechanical separator **40** contacts the fluid contained within the collection container **82,** air that occupies the through-hole **46** is progressively displaced by the fluid as the device submerges. When the mechanical separator **40** is submerged in the fluid, the float **42** has a greater buoyancy than the ballast **44,** which generates a differential force across the mechanical separator. During centrifugation, the differential force causes the float **42** component to elongate and contract away from the sidewall **86** of the collection container **82,** thereby reducing the effective diameter and opening a communicative pathway for the flow of fluid, such as higher and lower density phase components, past the separator body **41.** It is noted that the float **42** may be adapted for deformation in a direction substantially perpendicular to the through-hole **46.** As the applied rotational force is removed, the float **42** recovers and the sealing area defined by the float **42** and the first extended tab **52** and the second extended tab **54** re-expands to seal against the inner surface **94** of the collection container along a second sealing perimeter **106,** as shown in **FIG. 31****.** Accordingly, the mechanical separator **40** is adapted to prevent fluid from passing between or around the separator body **41** and the collection container **82,** and also prevents fluid from passing through the through-hole **46,** effectively establishing a barrier. The second sealing perimeter **106** establishes a barrier between higher and lower density phases within the sample.

As shown in **FIGS. 31A-31B****,** the mechanical separator **140A** includes a separator body **141A** having a float **142A** and a ballast **144A** with a through-hole **146A** defined within the float **142A,** as discussed above. In this configuration, the float **142A** may include a partially scalloped region **150A** for providing a surface to improve surface shedding of debris during use. As discussed herein, when the separator **140A** is submerged within a fluid sample, such as blood, certain blood constituents, such as fibrin or cells, may adhere to or become otherwise trapped on the upper surface of the float **142A.** In accordance with the present embodiment, the float **142A** may include a scalloped region **150A** for increasing the surface shedding. In another embodiment, the float **142A** may include opposing scalloped regions **150A,** such as shown in **FIG. 31B****.** The scalloped region **150A** may include any curved shape suitable to increase the surface shedding of the float, such as elliptical, oval, curved, and the like.

In this configuration, the separator body **141A** may also include the first extended tab **152A** and the second extended tab **154A** having enlarged fillets **180A** positioned at the edges of the first and second extended tabs **152A, 154A** adjacent the through-hole **146A** to assist in the formation of a barrier against a portion of the tube wall in the sealing position, as described herein. The enlarged fillets **180A** may include a region of the first and second extended tabs **152A, 154A** having an increased thickness and/or diameter, such as a widened taper adjacent the ends of the first and second extended tabs **152A, 154A** and extending along at least a portion of the through-hole **146A.** In one configuration, the enlarged fillets **180A** may facilitate shedding of cells around the mechanical separator body **141A** during application of applied rotational force, as described herein.

In accordance with a further embodiment of the present application, as shown in **FIGS. 31C-31I****,** the mechanical separator **40D** includes a separator body **41D** having a float **42D** and a ballast **44D** with a through-hole **46D** defined within the float **42D,** as discussed above. In this configuration, the separator body **41D** may have a substantially egg-shaped outer perimeter for improving the barrier seal between the mechanical separator **40D** and the sidewall of the collection container in the sealing position, such as is shown in **FIGS. 29** and **68****.**

In this configuration, the diameter **D₅** of the separator body **41D,** specifically the float **42D** as shown in **FIGS. 31D** and **31G****,** taken across the float **42D** in the direction along the through-axis **Tₐₓᵢₛ** of the through hole **46D,** as shown in **FIG. 31F****,** may be less than the diameter **D₆** of the separator body **41D,** specifically the float **42D** as shown in **FIG. 31D****,** taken across the float **42D** in the direction perpendicular to the through-axis **Tₐₓᵢₛ** of the through hole **46D,** as shown in **FIG. 31F****.** In this configuration, the diameter **D₇** of the separator body **41D,** specifically the float **42D** as shown in **FIG. 31D****,** taken across the float **42D** at an angle of 45° to the through-axis **Tₐₓᵢₛ** may be larger than the through-hole **46D,** or may be greater than the diameters **D₅** and **D₆** of the separator body **41D.** Also in this configuration, the diameter **D₈** of the ballast **44D** taken across the ballast **44D** along the through-axis **Tₐₓᵢₛ** of the through-hole **46D,** as shown in **FIG. 31F****,** may be less than any of the diameters **D_{5,} D₆**, or **D₇** of the separator body **41D.**

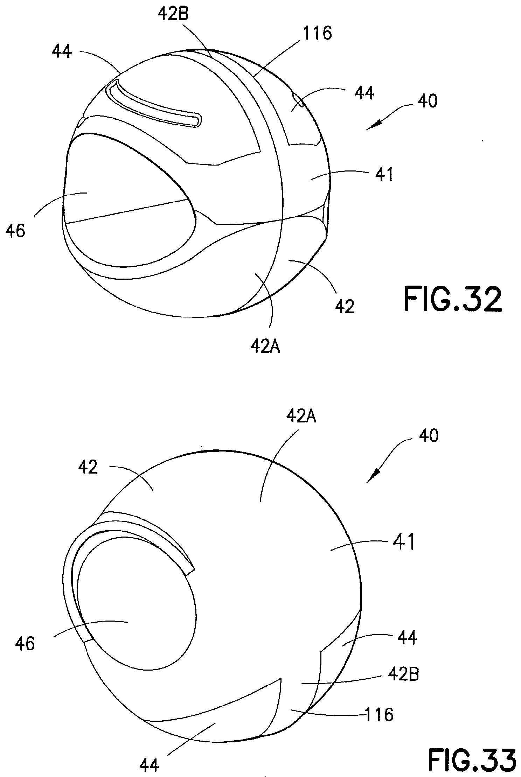

The provision of a float **42D** having an increased diameter with respect to the ballast **44D** may provide for a mechanical separator **40D** having an increased volume of lower density material, such as TPE, for displacing against a sealing surface as described herein. This embodiment may also include an extended tab band, as discussed below with respect to **FIGS. 35A-35E****,** and/or an initial engagement band, as discussed below with respect to **FIGS. 33-35****.**

Referring to **FIGS. 32-35****,** in a further configuration, the mechanical separator **40** may further include an initial engagement band **116** circumferentially disposed about the separator body **41.** In a further configuration, the initial engagement band **116** may be disposed about the separator body **41** in a direction substantially perpendicular to the through-hole **46.** The initial engagement band **116** may be continuously provided about the separator body **41,** or may optionally be provided in segments about the separator body **41.** In yet a further configuration, the float **42** and the initial engagement band **116** may be formed from the same material, such as TPE. The initial engagement band **116** may be provided such that a first portion **42A** of the float **42** forms the initial engagement band **116,** and a second portion **42B** substantially bisects the ballast **44.**

As shown specifically in **FIG. 35****,** the initial engagement band **116** provides an interference engagement between the separator body **41** and the inner surface **94** of the collection container **82.** In this configuration, a first sealing perimeter **104** about the separator body **41** is inline with the initial engagement band **116.** This first sealing perimeter **104** assists in maintaining the separator body **41** in proper alignment with the open top end **90** of the collection container **82,** such that fluid entering the collection container **82** from a cannula (not shown) disposed through the pierceable septum **100** will pass through the first opening **48** of the separator body **41,** through the through-hole **46,** and out the second opening **50.**

In accordance with yet another embodiment of the present invention, as shown in **FIGS. 35A-35E****,** the mechanical separator **40C** includes a separator body **41C** having a float **42C** and a ballast **44C.** The separator body **41C** includes a through-hole **46C** defined therein, such as defined entirely within the float **42C.** In this configuration, the **float 42C** may include an extended tab band **50C** disposed about an outer surface **52C** of the float **42C.** In one embodiment, the extended tab band **50C** may include a first extended portion **54C** adjacent a first opening **56C** of the through-hole **46C,** and a second extended portion **58C** adjacent the second opening **60C** of the through-hole **46C.** In this configuration, the first extended portion **54C** and the second extended portion **58C** may be provided substantially adjacent to at least a portion of the first opening **56C** and the second opening **60C,** respectively. The first extended portion **54C** and the second extended portion **58C** may each have a generally concave downwardly-directed orientation.

The first extended portion **54C** and the second extended portion **58C** may also be provided substantially about a portion of the through-hole **46C,** such as in an outwardly-extending arcuate shape about an upper portion of the through-hole **46C.** A portion of the first extended portion **54C** and a portion of the second extended portion **58C** may extend outwardly from the float **42C** in a direction substantially parallel to the through axis **T_{A}** of the separator body **41C,** such that the first extended portion **54C** and the second extended portion **58C** may have substantially the same shape and curvature.

The extended tab band **50C** may also include joining portions **62C** disposed between and connecting the first extended portion **54C** and the second extended portion **58C** on both sides of the separator body **41C.** The joining portions **62C** may each have a generally concave upwardly-directed orientation. In one embodiment, the joining portions **62C,** the first extended portion **54C,** and the second extended portion **58C** are continuous therewith, forming a generally "rope-like" appearance wrapped around a portion of the float **42C.** In a further embodiment, the joining portions **62C,** the first extended portion **54C,** and the second extended portion **58C** form a continuous sine function shape about a portion of the outer surface **52C** of the float **42C.** In another embodiment, the extended tab band **50C** may be co-formed with the float **42C,** forming a portion of the float **42C** itself. In an alternative embodiment, the extended tab band **50C** may be separately formed and subsequently joined with the float **42C.** In certain configurations, both the float **42C** and the extended tab band **50C** are made of a lower density material, such as TPE, and the ballast **44C** may be formed of a higher density material, such as PET.

In one embodiment, shown specifically in **FIGS. 35C** and **35C1**, the joining portions **62C** may each have approximately the same thickness **T_{J}**. In another embodiment, the first extended portion **54C** and the second extended portion **58C** may also have approximately the same thickness **T_{J}**. The cross-section of the extended tab band **50C** may have any suitable sealing shape such as rounded, squared, ribbed, or the like. It is also contemplated herein, that multiple extended tab bands **50C** may be disposed about the outer surface **52C** of the float **42C.** Referring to **FIGS. 35B** and **35D****,** the first extended portion **54C** and the second extended portion **58C** may include a thickened shelf region, **54C1** and **58C1,** respectively, defining a generally spline or saddle shape with the upper portion **64C** of the float **42C.** The upper portion **64C** of the float **42C** and the extended tab band **50C** may be particularly configured to maximize the surface shedding of debris during use. As discussed herein, when the separator **40C** is submerged within a fluid sample, such as blood, certain blood constituents, such as fibrin or cells, may adhere to or become otherwise trapped on the upper surface of the float **42C.** The specific shaping of the extended tab band **50C** is intended to minimize the trapping of debris during use.

In yet another embodiment, as shown in **FIG. 35E****,** the extended tab band **50C** may include a first extended portion **54C,** a second extended portion **58C,** and joining portions **62C** connecting the first extended portion **54C** and the second extended portion **58C** on both sides of the float **42C** so as to form a continuous structure about the outer surface **52C** of the float **42C.** In this configuration, the thickened shelf region **54C1** of the first extended portion **54C** and the thickened shelf region **58C1** of the second extended portion **58C** have a truncated profile **54C2** and **58C2,** respectively, to improve surface shedding of debris during use and to provide additional structural support to the first extended portion **54C** and the second extended portion **58C** during sealing with a collection container (not shown) in the sealing position.

When the mechanical separator **40C** of the present embodiment is in use, the extended tab band **50C** provides a robust sealing surface against a portion of the collection container wall (not shown), similar to the seal defined by the first extended tab and the second extended tab described above with reference to **FIGS. 1-8****.** In certain embodiments, the extended tab band **50C** may provide additional sealing and minimize leakage between the mechanical separator **40C** and the collection container. In addition, in the configurations in which the float **42C** is formed of TPE, the extended tab band **50C** provides a mechanism for enhanced sealing in that TPE does not appreciably deform under conventional applied rotational forces but rather displaces to another location. The location of the arcuate extended tab band **50C** about an outer surface **52C** of the float **42C** allows for the TPE to displace uniformly against a sidewall of the collection container in a sealing position, as described herein. As the extended tab band **50C** may be provided in an alternating concave upwardly-directed and concave downwardly-directed orientation, the sealing surface of the mechanical separator **40C** may be located at various heights about the outer surface **52C** of the float **42C** corresponding to the location of the extended tab band **50C.**

In an additional configuration, it is intended herein that the mechanical separator **40C** having an extended tab band **50C** may be suitable for use in collection containers having a tilted orientation due to the enhanced sealing between the extended tab band **50C** and the collection container (as described above) in the sealing position. It is also intended herein that the mechanical separator **40C** may include an initial engagement band **116,** as similarly described with reference to **FIG. 35** above.

In accordance with yet another embodiment of the present invention, as shown in **FIGS. 35F-35G****,** the mechanical separator **40A** includes a separator body **41A** having a float **42A** and a ballast **44A.** The separator body **41A** includes a through-hole **46A** defined therein. In this configuration, the ballast **44A** may include a base portion **52A** and a joining structure **48A,** such as a plurality of arms **50A** for engaging a portion of the float **42A.** The ballast **44A,** specifically the joining structure **48A,** may be provided in permanent engagement with a portion of the float **42A,** such as by co-molding, two-shot molding, welding, or other adhesive joining means. In one configuration, the float **42A** may be formed of a lower density material, such as TPE, and the ballast **44A** may be formed of a higher density material, such as PET. In a further configuration, the mechanical separator **40A** may be dimensioned such that the overall density of the separator body **41A is** between the density of higher and lower density constituents of a blood sample, such as serum and red blood cells. In yet a further embodiment, the overall density of the separator body **41A** is 1.45 g/cm³.

As shown in **FIG. 35H****,** the ballast **44A** may include a base portion **52A** having a contact surface **54A** and a joining surface **56A.** In one configuration, the contact surface **54A** may include an at least partially curved surface **58A** corresponding to an inner curvature of a collection container (not shown). The joining surface **56A** may include an attachment between the base portion **52A** and the joining structure **48A.** In one configuration, the joining surface **56A** and the joining structure **48A** are co-formed. In another configuration, the joining surface **56A** and the joining structure **48A** are separately formed and subsequently provided in permanent attachment through mechanical or adhesive locking means.

The joining structure **48A** may include a first end **60A** for engaging the base portion **52A** of the ballast **44A** and a second end **62A** for engaging a portion of the float **42A.** The top view of the float **42A** may have a substantially circular outer perimeter **P_{O}**, as shown in **FIG. 35I****,** and the float **42A** may have a substantially curved cross-sectional side view, such as a substantially concave down cross-section as shown in **FIG. 35H****.** In a further embodiment, the float **42A** may have a substantially concave down cross-section adjacent an apex **64A** of the float **42A,** and a slight concave upward curvature adjacent the perimeter **P_{O}** of the float **42A,** such as at a location at which the second end **62A** of the joining structure **48A** is attached to the float **42A.** In one configuration, the second end **62A** of the joining structure **48A** is molded first and the float **42A** is subsequently molded onto the second end **62A** of the joining structure **48A** to form a bond therewith. In another embodiment, the second end **62A** of the joining structure **48A** is inserted within, or provided adjacent to, a portion of the float **42A** and subsequently bonded or otherwise adhered thereto.

In one configuration, the joining structure **48A** may provide flexure between the float **42A** and the base portion **52A.** The flexure may be provided by at least one of the attachment between the first end **60A** of the joining structure **48A** and the base portion **52A,** the attachment between the second end **62A** of the joining structure **48A** and the float **42A,** and the pivot points **68A** of the joining structure **48A.**

Referring to **FIG. 35J****,** the mechanical separator **40A** may be provided within a collection container **100A,** such as adjacent an upper end **102A** of the collection container **100A** in an initial position. The mechanical separator **40A** may be provided in engagement with a portion of a stopper **104A,** such that a portion of the stopper **104A** extends through the through-hole **46A** of the mechanical separator **40A,** as described elsewhere herein. In accordance with another embodiment of the present invention, the mechanical separator **40A** may be provided such that a portion of the float **42A** and a portion of the base portion **52A** of the ballast **44A** engage an inner surface of the collection container **100A** to restrain the mechanical separator **40A** within the upper end **102A** of the collection container **100A** such that the through-hole **46A** of the mechanical separator **40A** is aligned with the longitudinal axis **L_{A}** of the collection container **100A.**

Referring again to **FIG. 35J****,** a fluid specimen **108A,** such as blood, is introduced into the collection container **100A,** such as through the stopper **104A** and aligned with through-hole **46A** of the mechanical separator **40A** when the mechanical separator **40A** is oriented in the initial position as shown by reference character **A.** As rotational force is applied, the float **42A** flexes and initiates a flexure between the float **42A** and the ballast **44A,** as described above. The resulting flexure deforms the through-hole **46A** and the mechanical separator **40A** disengages from the stopper **104A** and begins to rotate in the direction shown by arrow **R,** as shown by reference character **B.**

As the mechanical separator **40A** becomes submerged within the fluid specimen **108A,** the float **42A** begins to orient in an upward direction and the ballast **44A** simultaneously begins to orient in a downwards direction, as shown by reference character **C.** During the continued application of rotational force, the ballast **44A** pulls in a downwards direction and the float **42A** flexes away from the sidewall **110A** of the collection container, as shown by reference character **D.** Subsequently, as shown by reference character **E,** the float **42A** is deformed to allow for the passage of higher and lower density phase constituents between the float **42A** and the sidewall **110A** of the collection container **100A.** This allows for separation of the higher and lower density phase constituents within the fluid sample **108A,** as well as for the separation of higher and lower density phase constituents within the fluid sample **108A** present within the through-hole **46A** of the mechanical separator **40A.**

Referring to **FIG. 35K****,** once the application of rotational force has ceased, the mechanical separator **40A** becomes oriented between the separated higher density phase **112A** and the separated lower density phase **114A** in a sealing position. At the same time, the flexure between the float **42A** and the ballast **44A** ceases, causing the float **42A** to return to its initial position, as shown in **FIG. 35I****,** thereby forming a seal between the outer perimeter **P_{O}** and the interior circumference of the sidewall **110A** of the collection container 100A. The float **42A** has an outer perimeter **P_{O}** having an outer circumference that is at least slightly larger than the interior circumference of the sidewall **110A** of the collection container 100A, thereby forming a robust seal therebetween.

Referring yet again to **FIG. 35K****,** once the mechanical separator **40A** has been transitioned to the sealing position, a sealing perimeter is established along the outer perimeter **P_{O}** between at least a portion of the interior circumference of the sidewall **110A** and the mechanical separator **40A.** As shown in **FIG. 35K****,** the sealing perimeter along the outer perimeter **P_{O}** has a varying position about the interior circumference of the sidewall 110A as measured from the closed bottom end **113A** of the collection container **100A.** In one configuration, the sealing perimeter along the outer perimeter **P_{O}** includes various sealing heights at each localized sealing location, **S₁**, **S₂**, **S₃**, etc. corresponding to the overall height of the seal between the mechanical separator **40A,** specifically, the float **42A**, and the sidewall **110A**. The sealing perimeter accordingly has a height which varies slightly at each localized sealing location **S₁**, **S₂**, **S₃**, etc. The sealing perimeter also defines an average sealing height **H_{Avg}** which corresponds to the average height of each localized sealing location **S₁**, **S₂**, **S₃**, etc., i.e., **H_{Avg}** = **Avg** [**S₁**, **S₂**, **S₃**, etc.]. The mechanical separator **40A** also has a maximum height **H_{Max}** and a minimum height **H_{Min}** within the collection container. The maximum height **H_{Max}** corresponds to the distance between the highest seal point along the outer perimeter **P_{O}** and the closed bottom end **113A** of the collection container **100A.** The minimum height **H_{Min}** corresponds to the lowest seal point along the outer perimeter **P_{O}** and the closed bottom end **113A** of the collection container **100A.** In accordance with an aspect of the present invention, the average sealing height **H_{Avg}** is less than the difference between the maximum seal height **H_{Max}** and the minimum seal height **H_{Min}**, i.e., **H_{Avg}** < **H_{Max}** - **H_{Min}**.

In accordance with another embodiment of the present invention, as shown in **FIGS. 35L-35M****,** the mechanical separator **40B** includes a separator body **41B** having a float **42B** and a ballast **44B.** The separator body **41B** includes a through-hole **46B** defined therein. In this configuration, the float **42B** may include a joining structure **48B,** such as a plurality of arms **50B** for engaging a portion of the ballast **44B.** As similarly described above, the joining structure **48B** may be provided in permanent engagement with a portion of the ballast **44B,** such as by co-molding, two-shot molding, welding, or other adhesive joining means. In this configuration, the joining structure **48B** may exhibit increased flexibility allowing for easier transition from an initial position to a sealing position, as described herein.

Referring again to **FIGS. 35L-35M****,** in one configuration, the float **42B** may include a cut-out **60B** within the float **42B.** In one embodiment, the cut-out **60B** may be positioned at the apex **62B** of the float **42B** and does not extend into the outer perimeter **P_{O}.** The cut-out **60B** may provide for increased flexibility to allow passage of higher and lower density phase constituents thereby during use, such as shown in **FIG. 35J** with reference to reference character **E.** In yet a further configuration, the joining structure **48B** may include an opening **64B** therein adapted to allow a portion of the ballast **44B** to pass therethrough and be secured therein, such as by way of a mechanical interlock. In one embodiment, the joining structure **48B** includes a continuous arm **50B** connected to the float **42B** at a first end **68B** and a second end **70B.** The joining structure **48B** may include an opening **64B** having a locking portion **72B** of the ballast **44B** extending therethrough. In one embodiment, the opening **64B** may be disposed within the continuous arm **50B** at a location opposed from the apex **62B** of the float **42B.** In another embodiment, the ballast **44B,** such as the locking portion **72B,** and the float **42B** may be provided in permanent engagement so as to minimize separation of the float **42B** and the ballast **44B.**

Referring to **FIGS. 35N-35O**, in a further embodiment of the present invention, the mechanical separator **40B** includes a separator body **41B** having a float **42B** and a ballast **44B.** The separator body **41B** includes a through-hole **46B** defined therein. In this configuration, the float **42B** may include a joining structure **48B,** such as a plurality of arms **50B** for engaging a portion of the ballast **44B.** As similarly described above, the joining structure **48B** may include a continuous arm **50B** connected to the float **42B** at a first end **68B** and a second end **70B.** The joining structure **48B** may include an opening **64B** having a locking portion **72B** of the ballast **44B** extending therethrough in permanent engagement so as to minimize separation of the float **42B** and the ballast **44B.** The ballast **44B** may also include a support structure **74B** adjacent and connected to the joining structure **48B** of the float **42B.** In one embodiment, the support structure **74B** of the ballast **44B** may be co-formed or otherwise permanently engaged with the joining structure **48B** of the float **42B.** In a further embodiment, the joining structure **48B** may define a recess adapted to at least partially surround the support structure **74B.** In yet a further embodiment, the support structure **74B** and the joining structure **48B** allow the float **42B** and ballast **44B** to at least partially flex with respect to each other, as described herein. In certain configurations, a ballast cut-out **80B** may be provided within the base portion **52B** to lessen shrinkage of the ballast **44B** during formation.

Although the through-hole of the mechanical separator of the present invention has been shown herein as a straight bore having a spherical or elliptical cross-section, it is also contemplated herein that the through-hole **546,** as shown in **FIGS. 36-37****,** may define a serpentine or circuitous path for receiving liquid therethrough. In this configuration, the mechanical separator **540** includes a through-hole **546** having a first opening **549** and a second opening **551** that are offset with respect to each other. Specifically, the first opening **549** and the second opening **551** may be offset, such as at 60° or 90° angles with respect to each other. As shown in **FIG. 36****,** in the initial position, the first opening **549 is** aligned with the top open end **590** of the collection container **582,** represented herein in section. Fluid is directed through the through-hole **546** in the direction as shown by directional arrow **R.** In this configuration, at least one surface of the second opening **551** contacts the sidewall of the collection container **582,** while another surface of the second opening **551** remains free within the interior of the collection container **582.** Accordingly, a gap is provided between the sidewall of the collection container **582** and the second opening **551** of the through-hole **546** to allow fluid to exit the through-hole **546** and pass into the interior of the collection container **582.**

Upon application of rotational force, the mechanical separator **540** will transition from the initial position, as shown in **FIG. 36****,** to a sealing position, as shown in **FIG. 37****,** along directional arrow **S,** due to the moment of the float and ballast components as described herein. In this configuration, both the first opening **549** and the second opening **551** of the through-hole **546** are provided out of alignment with the top open end **590** of the collection container **582** and are adapted such that fluid is not directed into the through-hole **546.** A second sealing perimeter **595** is also established about the mechanical separator **540** such that fluid cannot pass between the mechanical separator **540** and the collection container **582** or through the through-hole **546** of the mechanical separator **540,** effectively establishing a barrier.

In yet another configuration, as shown in **FIGS. 38-39****,** the elongation of the mechanical separator **640** during application of rotational force is exemplified. In this configuration, the mechanical separator **640** may include a float **642** and a ballast **644** with a third section **643** joining the float **642** and the ballast **644.** It is contemplated herein, that in this configuration, both the float **642** and the ballast **644** may be made of a substantially rigid material with the float **642** having a density that is less than the density of the ballast **644.** In order to provide for an elongation between these components, the third section **643** formed of a flexible material, such as TPE, may be provided therebetween. During centrifugation, the third section **643** elongates, as shown in **FIG. 39****,** in a manner similarly described with respect to the elongation of the float above. During elongation of the third section **643,** higher and lower density phases of a fluid may pass adjacent the fluid passage surfaces **645,** as shown in **FIG. 39** as in a direction extending into the page.

With reference again to **FIG. 2** and **FIGS. 40** and **41,** the separator body **41** may include a center of mass **R** that is offset from the through-axis **T,** shown in **FIG. 2****,** of the separator body **41.** In this configuration, the mechanical separator **40** is transitionable from a first position (such as shown in **FIGS. 40-41**) in which the mechanical separator **40** is engaged with a portion of the closure **84** (shown in **FIG. 41****)** or a portion of the sidewall **86** of the collection container **82** (shown in **FIG. 40****)** and the center of mass **R** is oriented on a first side **S₁** of the longitudinal axis **L** of the collection container **82,** to a second position, such as shown in **FIG. 29****,** in which the mechanical separator **40** is disengaged from the closure or initial engagement position with the collection container, and the center of mass **R** is oriented across the longitudinal axis **L** of the collection container **82.** At some point, during the transition of the center of mass **R** across the longitudinal axis **L** of the collection container **82,** the float **42** of the mechanical separator **40** must deform in a direction substantially perpendicular to the through-axis T of the separator body **41** in order to allow for transition of the mechanical separator **40** from the initial first position to the second sealing position. During elongation of the float **42,** the higher and lower density phases of the specimen may pass between the mechanical separator **40,** specifically the elongated float **42,** and the sidewall **86** of the collection container **82** in which the mechanical separator is in an intermediate position. From the intermediate position, the mechanical separator may subsequently transition to the sealing position, in which a portion of the float 42 forms a sealing engagement with a portion of the interior of the collection container, upon termination of applied rotational force.

Accordingly, the mechanical separator of the present invention may be considered to transition between three phases of operation: the initial phase in which a specimen is provided through the through-hole of the separator body; the intermediate phase in which the separator has disengaged from the initial position and the float **42** is elongated to allow passage of higher and lower density phases thereby; and the sealing position in which the float **42** forms a barrier with a portion of the collection container. During this sequence of phases, the mechanical separator may be considered as "open-open-closed" wherein an "open" phase is defined as a state in which the mechanical separator does not form a sealing barrier with the collection container preventing the passage of fluid therethrough and therearound. In contrast, a "closed" phase is defined as a state in which mechanical separator 40 does form a sealing barrier with the collection container preventing the passage of fluid therethrough and therearound.

The mechanical separator of the present invention is also intended for use with various closure arrangements in the initial phase. Referring to **FIG. 40****,** the mechanical separator **40** may be maintained in the initial position by the interference between the float **42** and the initial engagement band **116** and the sidewall **86** of the collection container **82.** In this configuration, the mechanical separator **40** is not restrained by any portion of the closure **84.**

In another configuration, as shown in **FIGS. 41-44****,** the separation assembly includes a closure **84** and a post **180** engaged within a recess **181** of the closure **84.** The post **180** may include a separator receiving end **182** and a closure engagement end **183.** The closure engagement end **183** may be adapted for positioning within the recess **181** of the closure **84** and may optionally include at least one barb **184** for securing the post **180** within the closure **84.** The separator receiving end **182** may have any suitable profile such that it may be at least partially disposed within the through-hole **46** of the separator body **41.** In one embodiment, the separator receiving end **182** has a substantially circular cross-section. In another embodiment, the separator receiving end **182** has a substantially elliptical cross-section. The separator receiving end **182** is dimensioned to snugly fit within the through-hole **46** to provide a releaseable engagement with the mechanical separator **40.** The post **180** is also adapted for positioning within the interior of the collection container **82** and includes a post through-hole **186** aligned along the longitudinal axis of the collection container **82.** When the mechanical separator **40** is engaged with the post **180,** a fluid path is formed between the through-hole **46** of the mechanical separator **40** and the post through-hole **186** of the post **180.** This effectively forms a "sealed" fluid path for the direction of the fluid sample into the collection container **82.** Upon application of rotational force, the mechanical separator experiences a slight longitudinal movement prior to the axial rotation as the mechanical separator is pulled downward off the post **180** during applied rotation.

Referring to **FIGS. 45-46****,** an alternative separation assembly is shown including a collection container **782** having a first region **783** having an open top end **784** and a first sidewall **785** defining a first interior **786** and a first exterior **787.** The collection container **782** also includes a second region **788** having a closed bottom end **789** and a second sidewall **790** defining a second interior **791** and a second exterior **792.** In this configuration, the first region **783** and the second region **788** are aligned along a longitudinal axis **L_{A}** such that the first interior **786** and the second interior **791** are provided in fluid communication. The first interior **786** includes a first diameter **D_{F}** and the second interior **791** includes a second diameter **D_{S}**, with the first diameter **D_{F}** being greater than the second diameter **D_{S}**. The collection container **782** also includes at least one fluid flute **793** extending between the first region **783** and the second region **788** to allow passage of fluid therethrough from the first region **783** to the second region **788.** In this configuration, the first exterior **787** of the first region **783** may have a profile that corresponds to a 16 mm collection tube, and the second exterior **792** of the second region **788** may have a profile that corresponds to a 13 mm collection tube.

The first interior **786** of the first region **783** may be dimensioned to accommodate a mechanical separator **40** therein in any of the configurations described herein. The second interior **791** is dimensioned to at least partially restrain a portion of the mechanical separator **40** from passing therein in the initial position and absent applied rotational force. During application of rotational force, the float portion **42** of the mechanical separator **40** may elongate thereby decreasing the effective diameter of the mechanical separator **40** and allowing passage of the mechanical separator into the second interior **791.** In this configuration, the orientation of the through-hole **46** of the mechanical separator **40** is irrelevant as the introduction of fluid sample into the collection container **782** occurs around the separator body **41** as opposed to through the through-hole **46.** Specifically, fluid is introduced into the collection container **782** into the first interior **786** and around the mechanical separator **40.** The sample then passes into the second interior **791** by way of the fluid flutes **793.** Accordingly, the initial orientation of the mechanical separator **40** is irrelevant to the function of the separator in this embodiment.

In accordance with a further embodiment of the present invention, as shown in **FIG. 46A****,** a mechanical separator, as described herein, may be used with a collection container **782A** having a slight taper along a portion of the sidewall **783A** extending between an open top end **784A** and a closed bottom end **785A.** In this configuration, the collection container **782A** includes a first region indicator section **A** of **FIG. 46A****.** First region indicator section **A** is disposed along a portion of the sidewall **783A** at a distance **786A** from the open top end **784A.** The collection container **782A** may also include a second region indicator section **B** of **FIG. 46A****.** Second region indicator section **B** is disposed along a portion of the sidewall **783** at a distance **788A** from the open top end **784A.** In one configuration, the region defined between the first region indicator section **A** and the second region indicator **B** may have substantially no taper. In another configuration, the region defined between the first region indicator section **A** and the second region indicator **B** may have substantially may have a slight inward taper. In a further embodiment, the region defined between the first region indicator section **A** and the second region indicator **B** may be about the expected separation transition between the separated higher and lower density phases of a liquid to be separated.

In yet another embodiment, shown in **FIGS. 47-48****,** the separation assembly includes a closure **850** adapted for sealing engagement with the collection container **852.** The closure **850** includes a receiving end **842** for positioning within the open end **853** of the collection container **852.** The receiving end **842** defines an interior cavity **854** and includes an undercut protrusion **855** extending into the interior cavity **854.** The undercut protrusion **855** of the closure **850** is at least partially disposed within the through-hole **46** of the mechanical separator **40** in the initial position. Also in the initial position, at least a portion of the separator body **41** is disposed within the interior cavity **854.** The positioning of the mechanical separator **40** within the interior cavity **854** ensures that the mechanical separator **40** remains captured in the closure **850** during assembly of the closure **850** with the collection container **852.** This configuration may be utilized with the collection container having a first region and a second region, as described above. During application of rotational force, the float **42** of the mechanical separator **40** elongates allowing the mechanical separator **40** to disengage from the closure **850.**

Referring now to **FIGS. 49-59****,** various other engagements between the mechanical separator **40** and the closure **84** are also contemplated herein. As shown in **FIG. 49****,** the mechanical separator **40** may include an angled engagement boss **900** disposed within the through-hole **46** in the initial position. As shown in **FIG. 50****,** the mechanical separator **40** may include a substantially cylindrical engagement boss **901** disposed within the through-hole **46** in the initial position. A flanking portion **902** of the closure **903** may be provided adjacent an exterior surface **904** of the mechanical separator **40** adjacent the first opening **905** for further securing the mechanical separator **40** with the closure **903** and establishing a "sealed" fluid path into the collection container **906** therethrough.

Referring to **FIGS. 51-52****,** a sealant **907** may be provided adjacent the flanking portion **902,** as described above, for further securing the mechanical separator **40** and the closure **903.** The sealant **907** may be sufficiently tacky to retain the mechanical separator **40** in place in the initial position, yet weak enough to permit release of the mechanical separator **40** from the closure **903** upon application of rotational force.

Referring to **FIG. 53****,** yet another alternative angled engagement boss **908** may be disposed within the through-hole **46** in the initial position. Referring to **FIGS. 54-55****,** the closure **910** may include at least one, such as two, depending arms **911** for engagement with the mechanical separator **40.** In one configuration, each depending arm **911** includes a contact protrusion **912** for engaging a portion of the mechanical separator **40** within the through-hole **46** in the initial position. The interference between the contact protrusion **912** and the mechanical separator **40** may be sufficient to restrain the mechanical separator **40** with the closure **910** in the initial position, yet allow for disengagement of the mechanical separator **40** from the closure **910** upon application of rotational force.

Referring to **FIGS. 56-57****,** the closure **915** may include a molding insert **916** having a wedging basket **917** for further securing the molding insert **916** with the closure **915.** As described above, the molding insert **916** may include a separator receiving end **918** for engaging the mechanical separator **40** through the through-hole **46,** and a closure engagement end **919,** as described above. Referring to **FIG. 58****,** another molding insert **920** may include at least one barb **921** for further securing the molding insert **920** with the closure **922.** Referring to **FIG. 59****,** yet another molding insert **930** may include at least one protrusion **931** for securing the molding insert **930** with the closure **932.**

Referring to **FIGS. 60-68****,** the separation assemblies described herein may also include a carrier **650** releasably engaged with a portion of the mechanical separator **40** in the initial position. In each of these configurations, the carrier **650** disengages from the mechanical separator **40** upon application of rotational force and enters the fluid phase disposed below the mechanical separator **40** for the purpose of preventing clots or fibrin strands from interfering with the operation of the mechanical separator **40.**

As shown in **FIG. 60****,** the carrier **650** may include a closure engagement portion **651** for releasable engagement with a portion of the closure **652,** and a depending portion **653** for releasable engagement with a portion of the mechanical separator **40,** such as through the through-hole **46.** As shown in **FIG. 61****,** the carrier **650** may include a closure engagement portion **651** having a plurality of flanges **654.** The carrier **650** may also include a bowed separator engagement portion **655** for engaging a portion of the mechanical separator **40,** such as within the through-hole **46.** Upon application of rotational force, the mechanical separator **40** disengages from the initial position and rotates as described herein. Upon rotation of the mechanical separator **40,** the bowed separator engagement portion **655** contracts and allows the mechanical separator **40** to separate from the carrier **650.**

Referring to **FIGS. 63-66****,** the carrier **650** may also be releasably connected to the mechanical separator **40** in a direction opposed from the closure **660.** Referring to **FIGS. 67-****68,** the carrier **650** may optionally consist of a dissolvable material which diffuses into the sample when contact is made, as shown in **FIG. 68****.**

One of the significant benefits of the mechanical separator of the present invention is that it does not require penetration by a needle cannula in order to permit entry of a fluid sample into a collection container. In each of the above-described embodiments, when the assembly is subjected to an applied rotational force, such as centrifugation, the respective phases of the specimen, such as blood, will begin to separate into a denser phase displaced toward the bottom of the collection container, and a less dense phase displaced toward the top of the collection container. The applied rotational force will urge the ballast of the mechanical separator toward the closed bottom end and the float toward the top end of the collection container. This movement of the ballast will generate a longitudinal deformation of the float. As a result, the float will become longer and narrower and will be spaced concentrically inward from the inner surface of the cylindrical sidewall of the collection container. Accordingly, lighter phase components of the blood will be able to slide past the float and travel upwards, and likewise, heavier phase components of the blood will be able to slide past the float and travel downwards.

As noted above, the mechanical separator of the present invention typically has an overall density between the densities of the separated phases of the blood. Consequently, the mechanical separator will stabilize in a position within the collection container such that the heavier phase components will be located between the mechanical separator and the closed bottom end of the collection container, while the lighter phase components will be located between the mechanical separator and the top end of the collection container.

After this stabilized state has been reached, the centrifuge will be stopped and the float will resiliently return to its unbiased state and into sealing engagement with the interior of the cylindrical sidewall of the collection container. The formed liquid phases may then be accessed separately for analysis. In one embodiment, the assembled mechanical separator of the present invention may be scaled to fit within a 13 mm collection tube.

In use, the mechanical separator of the present invention minimizes device pre-launch and eliminates the need for cannula puncture which substantially eliminates sample pooling under the closure. Additionally, the reduced clearance of the mechanical separator minimizes the loss of trapped fluid phases, such as serum and plasma.

## Claims

1. A mechanical separator (40, 40B, 40C, 40D, 140, 140A, 240, 240A, 340, 440, 540) for separating a fluid into first and second phases within a collection container, comprising:
a separator body (41, 41C, 41D, 141, 141A, 241, 241A) comprising:
a float (42, 42B, 42C, 42D, 142, 142A, 242, 242A, 342, 442, 542), having a first density; and
a ballast (44, 44B, 44C, 44D, 144, 144A, 244, 244A, 344, 444, 544), having a second density greater than the first density, wherein a portion of the float (42, 42B, 42C, 42D, 142, 142A, 242, 242A, 342, 442, 542) is connected to a portion of the ballast (44, 44B, 44C, 44D, 144, 144A, 244, 244A, 344, 444, 544), and
wherein at least a portion of the separator body (41, 41B, 41C, 41D, 141, 141A, 241, 241A) is adapted for deformation upon applied rotational force
**characterized in that**
the separator body (41, 41C, 41D, 141, 141A, 241, 241A) has a through-hole (46, 46C, 46D, 146, 146A, 246, 246A, 346, 446, 546) defined therethrough, the through-hole (46, 42B, 46C, 46D, 146, 146A, 246, 246A, 346, 446, 546) comprising a first opening (48, 248, 56C, 549) and a second opening (80, 250, 60C, 551) and adapted to allow fluid to pass through the separator body (41, 41C, 41D, 141, 141A, 241, 241A), and
upon application of a rotational force to the collection container containing the separator (40, 40B, 40C, 40D, 140, 140A, 240, 240A, 340, 440, 540), the separator (40, 40B, 40C, 40D, 140, 140A, 240, 240A, 340, 440, 540) is adapted to rotate from a first initial position in which the through-hole (46, 42B, 46C, 46D, 146, 146A, 246, 246A, 346, 446, 546) is oriented in an open position for allowing fluid to pass through the through-hole (46, 42B, 46C, 46D, 146, 146A, 246, 246A, 346, 446, 546) and into the collection container to a second sealing position in which the separator body provides a seal with an inner surface of the collection container preventing fluid from being received through the through-hole (46, 42B, 46C, 46D, 146, 146A, 246, 246A, 346, 446, 546) and around the separator (40, 40B, 40C, 40D, 140, 140A, 240, 240A, 340, 440, 540).

2. The mechanical separator (40, 40C, 40D, 140, 140A, 240, 240A, 340, 440, 540) of claim 1, wherein at least a portion of the mechanical separator has a spheroid shape.

3. The mechanical separator (40, 40C, 40D, 140, 140A, 240, 240A, 340, 440, 540) of claim 2 or claim 3, wherein the float (42, 42C, 42D, 142, 142A, 242, 242A, 342, 442, 542) comprises an exterior surface (58) and a joining surface (60), and the ballast (44, 44C, 44D, 144, 144A, 244, 244A, 344, 444, 544) comprises a contact surface (64) connected to the joining surface (60) of the float (42, 42C, 42D, 142, 142A, 242, 242A, 342, 442, 542) and an exterior surface (62), wherein the exterior surface (58) of the float (42, 42C, 42D, 142, 142A, 242, 242A, 342, 442, 542) and the exterior surface (62) of the ballast (44, 44C, 44D, 144, 144A, 244, 244A, 344, 444, 544) taken together form the spheroid shape.

4. The mechanical separator (40, 40B, 40C, 40D, 140, 140A, 240, 240A, 340, 440, 540) of any of claims 1-3, wherein the float (42, 42B, 42C, 42D, 142, 142A, 242, 242A, 342, 442, 542) defines the through-hole (46, 46B, 46C, 46D, 146, 146A, 246, 246A, 346, 446, 546) adapted for allowing fluid to pass therethrough.

5. The mechanical separator (40, 40B, 40C, 40D, 140, 140A, 240, 240A, 340, 440, 540) of any of claims 1-4, wherein the through-hole (46, 46B, 46C, 46D, 146, 146A, 246, 246A, 346, 446, 546) is defined along a through-axis (T) and the float (42, 42B, 42C, 42D, 142, 142A, 242, 242A, 342, 442, 542) is adapted for deformation in a direction perpendicular to the through-axis (T) upon applied rotational force.

6. The mechanical separator (40, 40C, 40D, 140, 140A, 240, 240A, 340, 440, 540) of any of claims 1-3, wherein the float (42, 42C, 42D, 142, 142A, 242, 242A, 342, 442, 542) further comprises a first extended tab (52, 54C, 152, 152A, 252, 252A) adjacent the first opening (48, 56C) of the through-hole (46, 46C, 46D, 146, 146A, 246, 246A, 346, 446, 546) and a second extended tab (54, 58C, 154, 154A, 254, 254A) adjacent the second opening (50, 60C) of the through-hole (46, 46C, 46D, 146, 146A, 246, 246A, 346, 446, 546), wherein the first extended tab (52, 54C, 152, 152A, 252, 252A) and the second extended tab (54, 58C, 154, 154A, 254, 254A) are preferably provided at least partially above and about the through-hole (46, 46C, 46D, 146, 146A, 246, 246A, 346, 446, 546) and extend preferably radially outwardly from a portion of the float (42, 42C, 42D, 142, 142A, 242, 242A, 342, 442, 542) in a direction parallel to the through-axis (T) of the separator body (41, 41C, 41D, 141, 141A, 241, 241A).

7. The mechanical separator (40, 40C, 40D, 140, 140A, 240, 240A, 340, 440, 540) of claim 6, wherein the first extended tab (52, 54C, 152, 152A, 252, 252A), an upper surface of the float 42, 42C, 42D, 142, 142A, 242, 242A, 342, 442, 542), and the second extended tab (54, 58C, 154, 154A, 254, 254A) form an at least partially convex upper float surface.

8. The mechanical separator (40C) of any of claims 1-7, wherein the separator body (41C) an extended tab band (50C) disposed about a portion of an outer surface of the float (41C), wherein preferably a first extended portion (54C) of the extended tab band (50C) is disposed adjacent the first opening (56C) of the through-hole (46C) and a second extended portion (58C) of the extended tab band (50C) is disposed adjacent the second opening (60C) of the through-hole (46C).

9. The mechanical separator (40C) of claim 8, wherein at least one of the first extended portion (54C) and the second extended portion (58C) of the extended tab band (50C) have a concave downwardly-directed orientation and/or are oriented in an outwardly-extending arcuate shape about an upper portion of at least one of the first opening (56C) and second opening (60C) of the through-hole (60C).

10. The mechanical separator (40C) of claim 9, at least one of the first extended portion (54C) and the second extended portion (58C) of the extended tab band (50C) extend radially outwardly from the float (42C).

11. The mechanical separator (40C) of claim 9 or claim 10, wherein at least a portion of the first extended portion (54C) and at least a portion of the second extended portion (58C) of the extended tab band (50C) have the same shape and curvature.

12. The mechanical separator (40C) of any of claims 9-11, wherein the extended tab band (50C) further comprises a joining portion (62C) disposed between and connecting the first extended portion (54C) and the second extended portion (58C) disposed on each connecting side of the separator body (41C), wherein the first extended portion (54C) and the second extended portion (58C) of the extended tab band (50C) have a concave downwardly-directed orientation, and the joining portions (62C) of the extended tab band (50C) have a concave upwardly-directed orientation.

13. The mechanical separator (40C) of claim 8, wherein the float (42C) comprises the extended tab band (50C) and wherein preferably the float (42C) and the extended tab band (50C) are formed of TPE and the ballast (44C) is formed of PET.

14. The mechanical separator (40) of any of claims 1-13, further comprising an initial engagement band (116) circumferentially disposed about at least a portion of the separator body (41).

15. The mechanical separator (40) of claim 14, wherein the initial engagement band (116) is at least one of continuous and at least partially segmented and/or wherein the initial engagement band (116) and the float (42) are formed of the same material and/or wherein the initial engagement band (116) bisects at least a portion of the ballast (44).

16. The mechanical separator (40, 40B, 240) of any of claims 1-15, wherein the ballast (44, 44B, 244) comprises a base portion and a joining structure for engaging a portion of the float (42, 42B, 242).

17. The mechanical separator (40, 40B, 240) of claim 16, wherein the joining structure comprises a plurality of arms (50B) for engaging a portion of the float (42, 42B, 242) and/or wherein the joining structure provides flexure between the float (42, 42B, 242) and the ballast (44, 44B, 244).

18. The mechanical separator (40, 40B, 240) of any of claims 1-17, wherein the float (42, 42B, 242) comprises a joining structure for engaging a portion of the ballast (44, 44B, 244).

19. The mechanical separator (40, 40B, 240) of claim 18, wherein the joining structure comprises a plurality of arms (50B) for engaging a portion of the ballast (44, 44B, 244) and/or wherein the joining structure provides flexure between the float (42, 42B, 242) and the ballast (44, 44B, 244).

20. A separation assembly comprising the mechanical separator (40, 40B, 40C, 40D, 140, 140A, 240, 240A, 340, 440, 540) of any of claims 1-19; and a collection container (82) having a first end (90), a second end (88), and a sidewall (92) extending therebetween, the collection container (82) defining a longitudinal axis (L) between the first end (90) and the second end (88).

21. The separation assembly of claim 20, further comprising a closure (84) adapted for sealing engagement with the first end (90) of the collection container (82), wherein the mechanical separator (40, 40B, 40C, 40D, 140, 140A, 240, 240A, 340, 440, 540) is releasably engaged with a portion of the closure (84).

22. The separation assembly of claim 21, wherein the mechanical separator (40, 40B, 40C, 40D, 140, 140A, 240, 240A, 340, 440, 540) is engaged with a portion of the closure (84) in the first initial position and/or wherein the closure (84) further comprises an engagement boss (102) disposed within a portion of the through-hole (46, 42B, 46C, 46D, 146, 146A, 246, 246A, 346, 446, 546) when the separator body (41, 41B, 41C, 41D, 141, 141A, 241, 241A) is in the first initial position for forming a seal between a portion of the separator body (41, 41B, 41C, 41D, 141, 141A, 241, 241A) and the closure (84).

23. The separation assembly of any of claims 20-22, wherein at least a portion of the through-hole (46, 42B, 46C, 46D, 146, 146A, 246, 246A, 346, 446, 546) is oriented along the longitudinal axis (L) of the collection container (82) in the first initial position, and wherein at least a portion of the through-hole (46, 42B, 46C, 46D, 146, 146A, 246, 246A, 346, 446, 546) is oriented perpendicular to the longitudinal axis (L) of the collection container (82) in the second sealing position.

24. The mechanical separator (40, 40B, 240) of any of claims 1-19, wherein at least a portion of the float (42, 42B, 242) comprises a circular outer perimeter having a curved cross-section perpendicular to the through-hole (46, 46B, 246), wherein the outer perimeter of the float (42, 42B, 242) forms a continuous seal with the inner surface of the collection container (82) in the second sealing position.

## Patentansprüche

1. Mechanischer Separator (40, 40B, 40C, 40D, 140, 140A, 240, 240A, 340, 440, 540) zum Separieren eines Fluids in eine erste und eine zweite Phase innerhalb eines Auffangbehälters, mit:
einem Separatorkörper (41, 41C, 41D, 141, 141A, 241, 241A) mit:
einem Schwimmer (42, 42B, 42C, 42D, 142, 142A, 242, 242A, 342, 442, 542) mit einer ersten Dichte; und
einem Ballast (44, 44B, 44C, 44D, 144, 144A, 244, 244A, 344, 444, 544) mit einer zweiten Dichte, die größer als die erste Dichte ist, wobei ein Teil des Schwimmers mit einem Teil des Ballasts (44, 44B, 44C, 44D, 144, 144A, 244, 244A, 344, 444, 544) verbunden ist, und
wobei zumindest ein Teil des Separatorkörpers (41, 41C, 41D, 141, 141A, 241, 241A) zur Verformung beim Aufbringen einer Drehkraft geeignet ist,
**dadurch gekennzeichnet, dass**
der Separatorkörper (41, 41C, 41D, 141, 141A, 241, 241A) ein durch diesen gebildetes Durchgangsloch (46, 46C, 46D, 146, 146A, 246, 246A, 346, 446, 546), wobei das Durchgangsloch (46, 46C, 46D, 146, 146A, 246, 246A, 346, 446, 546) eine erste Öffnung (48, 248, 56C, 549) und eine zweite Öffnung (80, 250, 60C, 551) aufweist und geeignet ist, den Durchtritt von Fluid durch den Separatorkörper (41, 41C, 41D, 141, 141A, 241, 241A) zu ermöglichen, und
der Separator (40, 40B, 40C, 40D, 140, 140A, 240, 240A, 340, 440, 540) geeignet ist, sich beim Aufbringen einer Drehkraft auf den den Separator (40, 40B, 40C, 40D, 140, 140A, 240, 240A, 340, 440, 540) enthaltenden Sammelbehälter aus einer ersten Ausgangsposition, in welcher das Durchgangsloch (46, 46C, 46D, 146, 146A, 246, 246A, 346, 446, 546) in einer offenen Position für den Durchtritt von Fluid durch das Durchgangsloch (46, 46C, 46D, 146, 146A, 246, 246A, 346, 446, 546) und in den Sammelbehälter ausgerichtet ist, und in eine zweite Dichtposition zu drehen, in welcher der Separatorkörper eine Dichtung mit einer Innenfläche des Sammelbehälters bildet, welche das Aufnehmen von Fluid durch das Durchgangsloch (46, 46C, 46D, 146, 146A, 246, 246A, 346, 446, 546) und um den Separator (40, 40B, 40C, 40D, 140, 140A, 240, 240A, 340, 440, 540) verhindert.

2. Mechanischer Separator (40, 40B, 40C, 40D, 140, 140A, 240, 240A, 340, 440, 540) nach Anspruch 1, bei welchem mindestens ein Teil des mechanischen Separators eine Kugelform aufweist.

3. Mechanischer Separator (40, 40B, 40C, 40D, 140, 140A, 240, 240A, 340, 440, 540) nach Anspruch 2 oder 3, bei welchem der Schwimmer (42, 42B, 42C, 42D, 142, 142A, 242, 242A, 342, 442, 542) eine Außenfläche (58) und eine Verbindungsfläche (60) aufweist und der Ballast (44, 44B, 44C, 44D, 144, 144A, 244, 244A, 344, 444, 544) eine Kontaktfläche (64), die mit der Verbindungsfläche (60) des Schwimmers (42, 42B, 42C, 42D, 142, 142A, 242, 242A, 342, 442, 542) verbunden ist, und eine Außenfläche (62) aufweist, wobei die Außenfläche (58) des Schwimmers (42, 42B, 42C, 42D, 142, 142A, 242, 242A, 342, 442, 542) und die Außenfläche (62) des Ballasts (44, 44B, 44C, 44D, 144, 144A, 244, 244A, 344, 444, 544) zusammen die Kugelform bilden.

4. Mechanischer Separator (40, 40B, 40C, 40D, 140, 140A, 240, 240A, 340, 440, 540) nach einem der Ansprüche 1-3, bei welcher der Schwimmer (42, 42B, 42C, 42D, 142, 142A, 242, 242A, 342, 442, 542) das Durchgangsloch (46, 46C, 46D, 146, 146A, 246, 246A, 346, 446, 546) definiert, das geeignet ist, den Durchtritt von Fluid zu ermöglichen.

5. Mechanischer Separator (40, 40B, 40C, 40D, 140, 140A, 240, 240A, 340, 440, 540) nach einem der Ansprüche 1-4, bei welchem das Durchgangsloch (46, 46C, 46D, 146, 146A, 246, 246A, 346, 446, 546) entlang einer Durchgangsachse (T) ausgebildet ist und der Schwimmer (42, 42B, 42C, 42D, 142, 142A, 242, 242A, 342, 442, 542) beim Aufbringen einer Rotationskraft zur Verformung in einer Richtung senkrecht zur Durchgangsachse (T) geeignet ist.

6. Mechanischer Separator (40, 40B, 40C, 40D, 140, 140A, 240, 240A, 340, 440, 540) nach Anspruch 1-3, bei welchem der Schwimmer (42, 42B, 42C, 42D, 142, 142A, 242, 242A, 342, 442, 542) ferner eine erste verlängerte Lasche (52, 54C, 153, 152A, 252, 252A) neben der ersten Öffnung (48, 56C) des Durchgangslochs (46, 46C, 46D, 146, 146A, 246, 246A, 346, 446, 546) und eine zweite verlängerte Lasche (54, 58C, 154, 154A, 254, 254A) neben der zweiten Öffnung des Durchgangslochs (46, 46C, 46D, 146, 146A, 246, 246A, 346, 446, 546) aufweist, wobei die erste verlängerte Lasche (52, 54C, 153, 152A, 252, 252A) und die zweite verlängerte Lasche (54, 58C, 154, 154A, 254, 254A) vorzugsweise mindestens teilweise über und um das Durchgangsloch (46, 46C, 46D, 146, 146A, 246, 246A, 346, 446, 546) vorgesehen sind und sich vorzugsweise von einem Teil des Schwimmers (42, 42B, 42C, 42D, 142, 142A, 242, 242A, 342, 442, 542) radial nach außen in einer Richtung parallel zur Durchgangsachse (T) des Separatorkörpers (41, 41C, 41D, 141, 141A, 241, 241A) erstrecken.

7. Mechanischer Separator (40, 40B, 40C, 40D, 140, 140A, 240, 240A, 340, 440, 540) nach Anspruch 6, bei welchem die erste verlängerte Lasche (52, 54C, 153, 152A, 252, 252A), eine Oberseite des Schwimmers (42, 42B, 42C, 42D, 142, 142A, 242, 242A, 342, 442, 542) und die zweite verlängerte Lasche (54, 58C, 154, 154A, 254, 254A) eine zumindest teilweise konvexe Schwimmeroberseite bilden.

8. Mechanischer Separator (40C) nach einem der Ansprüche 1-7, bei welchem der Separatorkörper (41C) ferner ein verlängertes Laschenband (50C) aufweist, das um einen Teil einer Außenfläche des Schwimmers (42C) angeordnet ist, wobei vorzugsweise ein erster verlängerter Bereich (54C) des verlängerten Laschenbands (50C) neben der ersten Öffnung (56C) des Durchgangslochs (46C) angeordnet ist und ein zweiter verlängerter Bereich (58C) des verlängerten Laschenbands (50C) neben der zweiten Öffnung (60C) des Durchgangslochs (46C) angeordnet ist.

9. Mechanischer Separator (40C) nach Anspruch 8, bei welchem der erste verlängerte Bereich (54C) und/oder der zweite verlängerte Bereich (58C) des verlängerten Laschenbands (50C) eine konkave nach unten gerichtete Ausrichtung aufweist und/oder in einer sich nach außen erstreckenden bogenförmigen Form um einen oberen Bereich der ersten Öffnung (56C) und/oder der zweiten Öffnung (60C) des Durchgangslochs (46C) ausgerichtet ist.

10. Mechanischer Separator (40C) nach Anspruch 9, bei welchen sich der erste verlängerte Bereich (54C) und/oder der zweite verlängerte Bereich (58C) des verlängerten Laschenbands (50C) von dem Schwimmer (42C) radial nach außen erstreckt.

11. Mechanischer Separator (40C) nach Anspruch 9 oder Anspruch 10, bei welchem mindestens ein Teil des ersten verlängerten Bereichs (54C) und mindestens ein Teil des zweiten verlängerten Bereichs (58C) des verlängerten Laschenbands (5oC) die gleiche Form und Krümmung aufweisen.

12. Mechanischer Separator (40C) nach einem der Ansprüche 9-11, bei welchem das verlängerte Laschenband (50C) ferner einen Verbindungsbereich (62C) aufweist, der zwischen dem ersten verlängerten Bereich (54C) und dem zweiten verlängerten Bereich (58C) angeordnet ist und diese verbindet, wobei der Verbindungsbereich auf jeder Verbindungsseite des Separatorkörpers (41C) angeordnet ist, wobei der erste verlängerte Bereich (54C) und der zweite verlängerte Bereich (58C) des verlängerten Laschenbands (50C) eine konkave nach unten gerichtete Ausrichtung aufweisen und die Verbindungsbereiche (62C) des verlängerten Laschenbands (50C) eine konkave nach oben gerichtete Ausrichtung aufweisen.

13. Mechanischer Separator (40C) nach Anspruch 8, bei welchem der Schwimmer (42C) das verlängerte Laschenband (50C) aufweist, und wobei vorzugsweise der Schwimmer (42C) und das verlängerte Laschenband (50C) aus TPE bestehen und der Ballast (44C) aus PET besteht.

14. Mechanischer Separator (40) nach einem der Ansprüche 1-13, ferner mit einem Anfangseingriffsband (116), das in Umfangsrichtung um mindestens einen Teil des Separatorkörpers (41) angeordnet ist.

15. Mechanischer Separator (40) nach Anspruch 14, bei welchem das Anfangseingriffsband (116) durchgehend und/oder zumindest teilweise segmentiert ist, und/oder bei welchem das Anfangseingriffsband (116) und der Schwimmer (42) aus demselben Material gebildet sind, und/oder bei welchem das Anfangseingriffsband zumindest einen Teil des Ballasts (44) halbiert.

16. Mechanischer Separator (40, 40B, 240) nach einem der Ansprüche 1-15, bei welchem der Ballast (44, 44B, 244) einen Basisbereich und eine Verbindungsstruktur zum Zusammengreifen mit einem Bereich des Schwimmers (42, 42B, 242) aufweist.

17. Mechanischer Separator (40, 40B, 240) nach Anspruch 16, bei welchem die Verbindungsstruktur mehrere Arme (50B) zum Zusammengreifen mit einem Teil des Schwimmers (42, 42B, 242) aufweist.

18. Mechanischer Separator (40, 40B, 240) nach einem der Ansprüche 1-17, bei welchem der Schwimmer (42, 42B, 242) eine Verbindungsstruktur zum Zusammengreifen mit einem Bereich des Ballasts (44, 44B, 244) aufweist.

19. Mechanischer Separator (40, 40B, 240) nach Anspruch 18, bei welchem die Verbindungsstruktur mehrere Arme (50B) zum Zusammengreifen mit einem Bereich des Ballasts (44, 44B, 244) aufweist und/oder bei welchem die Verbindungsstruktur ein Biegen zwischen dem Schwimmer (42, 42B, 242) und dem Ballast (44, 44B, 144) ermöglicht.

20. Separationsanordnung mit dem mechanischen Separator (40, 40B, 40C, 40D, 140, 140A, 240, 240A, 340, 440, 540) nach einem der Ansprüche 1-19, und einem Sammelbehälter (82) mit einem ersten Ende (90), einem zweiten Ende (88) und einer sich zwischen diesen erstreckenden Seitenwände (92), wobei der Sammelbehälter (82) eine Längsachse (L) zwischen dem ersten Ende (90) und dem zweiten Ende (88) definiert.

21. Separationsanordnung nach Anspruch 20, ferner mit einem Verschluss (84), der zum dichtenden Zusammengreifen mit dem ersten Ende (90) des Sammelbehälters (82) geeignet ist, wobei der mechanische Separator (40, 40B, 40C, 40D, 140, 140A, 240, 240A, 340, 440, 540) lösbar mit einem Teil des Verschlusses (84) zusammengreift.

22. Separationsanordnung nach Anspruch 21, bei welcher der mechanische Separator (40, 40B, 40C, 40D, 140, 140A, 240, 240A, 340, 440, 540) mit einem Bereich des Verschlusses (84) in der ersten Ausgangsposition zusammengreift und/oder bei welcher der Verschluss (84) ferner einen Eingriffsvorsprung (102) aufweist, der in einem Bereich des Durchgangslochs (46, 46C, 46D, 146, 146A, 246, 246A, 346, 446, 546) angeordnet ist, wenn sich der Separatorkörper (41, 41C, 41D, 141, 141A, 241, 241A) in der ersten Ausgangsposition befindet, um eine Dichtung zwischen einem Bereich des Separatorkörpers (41, 41C, 41D, 141, 141A, 241, 241A) und dem Verschluss (84) zu bilden.

23. Separationsanordnung nach einem der Ansprüche 20-22, bei welcher mindestens ein Teil des Durchgangslochs (46, 46C, 46D, 146, 146A, 246, 246A, 346, 446, 546) in der ersten Ausgangsposition entlang der Längsachse (L) des Sammelbehälters (82) ausgerichtet ist, und bei welcher mindestens ein Teil des Durchgangslochs (46, 46C, 46D, 146, 146A, 246, 246A, 346, 446, 546) in der zweiten Dichtposition senkrecht zu der Längsachse (L) des Sammelbehälters (82) ausgerichtet ist.

24. Mechanischer Separator (40, 40B, 240) nach einem der Ansprüche 1-19, bei welchem mindestens ein Bereich des Schwimmers (42, 42B, 242) einen kreisförmigen Außenumfang mit einem gebogenen Querschnitt senkrecht zu dem Durchgangsloch (46, 46B, 246) aufweist, wobei der Außenumfang des Schwimmers (42, 42B, 242) in der zweiten Dichtposition eine durchgehende Dichtung mit der Innenfläche des Sammelbehälters (82) bildet.

## Revendications

1. Séparateur mécanique (40, 40B, 40C, 40D, 140, 140A, 240, 240A, 340, 440, 540) pour séparer un fluide en des première et deuxième phases à l'intérieur d'un récipient de collecte, comprenant :
un corps de séparateur (41, 41C, 41D, 141, 141A, 241, 241A) comprenant :
un flotteur (42, 42B, 42C, 42D, 142, 142A, 242, 242A, 342, 442, 542), ayant une première densité ; et
un ballast (44, 44B, 44C, 44D, 144, 144A, 244, 244A, 344, 444, 544), ayant une deuxième densité supérieure à la première densité, où une partie du flotteur (42, 42B, 42C, 42D, 142, 142A, 242, 242A, 342, 442, 542) est reliée à une partie du ballast (44, 44B, 44C, 44D, 144, 144A, 244, 244A, 344, 444, 544), et
dans lequel au moins une partie du corps de séparateur (41, 41B, 41C, 41D, 141, 141A, 241, 241A) est adaptée pour une déformation suite à l'application d'une force de rotation,
**caractérisé en ce que**
le corps de séparateur (41, 41C, 41D, 141, 141A, 241, 241A) a un trou traversant (46, 46C, 46D, 146, 146A, 246, 246A, 346, 446, 546) défini à travers celui-ci, le trou traversant (46, 42B, 46C, 46D, 146, 146A, 246, 246A, 346, 446, 546) comprenant une première ouverture (48, 248, 56C, 549) et une deuxième ouverture (80, 250, 60C, 551) et adapté pour permettre au fluide de passer à travers le corps de séparateur (41, 41C, 41D, 141, 141A, 241, 241A), et
lors de l'application d'une force de rotation sur le récipient de collecte contenant le séparateur (40, 40B, 40C, 40D, 140, 140A, 240, 240A, 340, 440, 540), le séparateur (40, 40B, 40C, 40D, 140, 140A, 240, 240A, 340, 440, 540) est adapté pour tourner d'une première position initiale dans laquelle le trou traversant (46, 42B, 46C, 46D, 146, 146A, 246, 246A, 346, 446, 546) est orienté dans une position ouverte pour permettre au fluide de passer à travers le trou traversant (46, 42B, 46C, 46D, 146, 146A, 246, 246A, 346, 446, 546) et dans le récipient de collecte vers une deuxième position d'étanchéité dans laquelle le corps de séparateur fournit un joint d'étanchéité avec une surface intérieure du récipient de collecte empêchant le fluide d'être reçu à travers le trou traversant (46, 42B, 46C, 46D, 146, 146A, 246, 246A, 346, 446, 546) et autour du séparateur (40, 40B, 40C, 40D, 140, 140A, 240, 240A, 340, 440, 540).

2. Séparateur mécanique (40, 40C, 40D, 140, 140A, 240, 240A, 340, 440, 540) de la revendication 1, dans lequel au moins une partie du séparateur mécanique a une forme sphéroïde.

3. Séparateur mécanique (40, 40C, 40D, 140, 140A, 240, 240A, 340, 440, 540) de la revendication 2 ou 3, dans lequel le flotteur (42, 42C, 42D, 142, 142A, 242, 242A, 342, 442, 542) comprend une surface extérieure (58) et une surface de jonction (60), et le ballast (44, 44C, 44D, 144, 144A, 244, 244A, 344, 444, 544) comprend une surface de contact (64) reliée à la surface de jonction (60) du flotteur (42, 42C, 42D, 142, 142A, 242, 242A, 342, 442, 542) et une surface extérieure (62), où la surface extérieure (58) du flotteur (42, 42C, 42D, 142, 142A, 242, 242A, 342, 442, 542) et la surface extérieure (62) du ballast (44, 44C, 44D, 144, 144A, 244, 244A, 344, 444, 544) constituent ensemble la forme sphéroïde.

4. Séparateur mécanique (40, 40B, 40C, 40D, 140, 140A, 240, 240A, 340, 440, 540) de l'une des revendications 1 à 3, dans lequel le flotteur (42, 42B, 42C, 42D, 142, 142A, 242, 242A, 342, 442, 542) définit le trou traversant (46, 46B, 46C, 46D, 146, 146A, 246, 246A, 346, 446, 546) adapté pour permettre au fluide de passer à travers celui-ci.

5. Séparateur mécanique (40, 40B, 40C, 40D, 140, 140A, 240, 240A, 340, 440, 540) de l'une des revendications 1 à 4, dans lequel le trou traversant (46, 46B, 46C, 46D, 146, 146A, 246, 246A, 346, 446, 546) est défini le long d'un axe traversant (T) et le flotteur (42, 42B, 42C, 42D, 142, 142A, 242, 242A, 342, 442, 542) est adapté pour une déformation dans une direction perpendiculaire à l'axe traversant (T) lors de l'application d'une force de rotation.

6. Séparateur mécanique (40, 40C, 40D, 140, 140A, 240, 240A, 340, 440, 540) de l'une des revendications 1 à 3, dans lequel le flotteur (42, 42C, 42D, 142, 142A, 242, 242A, 342, 442, 542) comprend en outre une première languette étendue (52, 54C, 152, 152A, 252, 252A) adjacente à la première ouverture (48, 56C) du trou traversant (46, 46C, 46D, 146, 146A, 246, 246A, 346, 446, 546) et une deuxième languette étendue (54, 58C, 154, 154A, 254, 254A) adjacente à la deuxième ouverture (50, 60C) du trou traversant (46, 46C, 46D, 146, 146A, 246, 246A, 346, 446, 546), où la première languette étendue (52, 54C, 152, 152A, 252, 252A) et la deuxième languette étendue (54, 58C, 154, 154A, 254, 254A) sont de préférence prévues au moins partiellement au-dessus et autour du trou traversant (46, 46C, 46D, 146, 146A, 246, 246A, 346, 446, 546) et s'étendent de préférence radialement vers l'extérieur à partir d'une partie du flotteur (42, 42C, 42D, 142, 142A, 242, 242A, 342, 442, 542) dans une direction parallèle à l'axe traversant (T) du corps de séparateur (41, 41C, 41D, 141, 141A, 241, 241A).

7. Séparateur mécanique (40, 40C, 40D, 140, 140A, 240, 240A, 340, 440, 540) de la revendication 6, dans lequel la première languette étendue (52, 54C, 152, 152A, 252, 252A), une surface supérieure du flotteur (42, 42C, 42D, 142, 142A, 242, 242A, 342, 442, 542), et la deuxième languette étendue (54, 58C, 154, 154A, 254, 254A) constituent une surface supérieure de flotteur au moins partiellement convexe.

8. Séparateur mécanique (40C) de l'une des revendications 1 à 7, dans lequel le corps de séparateur (41C) est une bande de languette étendue (50C) disposée autour d'une partie d'une surface extérieure du flotteur (41C),
où de préférence une première partie étendue (54C) de la bande de languette étendue (50C) est disposée de manière adjacente à la première ouverture (56C) du trou traversant (46C) et une deuxième partie étendue (58C) de la bande de languette étendue (50C) est disposé de manière adjacente à la deuxième ouverture (60C) du trou traversant (46C).

9. Séparateur mécanique (40C) de la revendication 8, dans lequel au moins l'une de la première partie étendue (54C) et de la deuxième partie étendue (58C) de la bande de languette étendue (50C) a une orientation concave dirigée vers le bas et/ou est orientée selon une forme arquée s'étendant vers l'extérieur autour d'une partie supérieure d'au moins l'une de la première ouverture (56C) et de la deuxième ouverture (60C) du trou traversant (60C).

10. Séparateur mécanique (40C) de la revendication 9, au moins l'une de la première partie étendue (54C) et de la deuxième partie étendue (58C) de la bande de languette étendue (50C) s'étend radialement vers l'extérieur depuis le flotteur (42C).

11. Séparateur mécanique (40C) de la revendication 9 ou 10, dans lequel au moins une partie de la première partie étendue (54C) et au moins une partie de la deuxième partie étendue (58C) de la bande de languette étendue (50C) ont la même forme et la même courbure.

12. Séparateur mécanique (40C) de l'une des revendications 9 à 11, dans lequel la bande de languette étendue (50C) comprend en outre une partie de jonction (62C) disposée entre et reliant la première partie étendue (54C) et la deuxième partie étendue (58C) disposées sur chaque côté de liaison du corps de séparateur (41C), où la première partie étendue (54C) et la deuxième partie étendue (58C) de la bande de languette étendue (50C) ont une orientation concave dirigée vers le bas, et les parties de jonction (62C) de la bande de languette étendue (50C) ont une orientation concave dirigée vers le haut.

13. Séparateur mécanique (40C) de la revendication 8, dans lequel le flotteur (42C) comprend la bande de languette étendue (50C) et dans lequel de préférence le flotteur (42C) et la bande de languette étendue (50C) sont formés à partir d'élastomères thermoplastiques (TPE) et le ballast (44C) est formé à partir de polyéthylène téréphtalate (PET).

14. Séparateur mécanique (40) de l'une des revendications 1 à 13, comprenant en outre une bande d'engagement initial (116) disposée de manière circonférentielle autour d'au moins une partie du corps de séparateur (41).

15. Séparateur mécanique (40) de la revendication 14, dans lequel la bande d'engagement initial (116) est au moins l'une d'une bande continue et d'une bande au moins partiellement segmentée et/ou dans lequel la bande d'engagement initial (116) et le flotteur (42) sont formés du même matériau et/ou dans lequel la bande d'engagement initial (116) bissecte au moins une partie du ballast (44).

16. Séparateur mécanique (40, 40B, 240) de l'une des revendications 1 à 15, dans lequel le ballast (44, 44B, 244) comprend une partie de base et une structure de jonction pour s'engager avec une partie du flotteur (42, 42B, 242).

17. Séparateur mécanique (40, 40B, 240) de la revendication 16, dans lequel la structure de jonction comprend une pluralité de bras (50B) pour s'engager avec une partie du flotteur (42, 42B, 242) et/ou dans lequel la structure de jonction fournit une flexure entre le flotteur (42, 42B, 242) et le ballast (44, 44B, 244).

18. Séparateur mécanique (40, 40B, 240) de l'une des revendications 1 à 17, dans lequel le flotteur (42, 42B, 242) comprend une structure de jonction pour s'engager avec une partie du ballast (44, 44B, 244).

19. Séparateur mécanique (40, 40B, 240) de la revendication 18, dans lequel la structure de jonction comprend une pluralité de bras (50B) pour s'engager avec une partie du ballast (44, 44B, 244) et/ou dans lequel la structure de jonction fournit une flexure entre le flotteur (42, 42B, 242) et le ballast (44, 44B, 244).

20. Ensemble de séparation comprenant le séparateur mécanique (40, 40B, 40C, 40D, 140, 140A, 240, 240A, 340, 440, 540) de l'une des revendications 1 à 19 ; et un récipient de collecte (82) ayant une première extrémité (90), une deuxième extrémité (88) et une paroi latérale (92) s'étendant entre celles-ci, le récipient de collecte (82) définissant un axe longitudinal (L) entre la première extrémité (90) et la deuxième extrémité (88).

21. Ensemble de séparation de la revendication 20, comprenant en outre une fermeture (84) adaptée pour un engagement étanche avec la première extrémité (90) du récipient de collecte (82), où le séparateur mécanique (40, 40B, 40C, 40D, 140, 140A, 240, 240A, 340, 440, 540) est engagé de manière amovible avec une partie de la fermeture (84).

22. Ensemble de séparation selon la revendication 21, dans lequel le séparateur mécanique (40, 40B, 40C, 40D, 140, 140A, 240, 240A, 340, 440, 540) est engagé avec une partie de la fermeture (84) dans la première position initiale et/ou dans lequel la fermeture (84) comprend en outre un bossage d'engagement (102) disposé dans une partie du trou traversant (46, 42B, 46C, 46D, 146, 146A, 246, 246A, 346, 446, 546) lorsque le corps de séparateur (41, 41B, 41C, 41D, 141, 141A, 241, 241A) est dans la première position initiale pour former un joint d'étanchéité entre une partie du corps de séparateur (41, 41B, 41C, 41D, 141, 141A, 241, 241A) et la fermeture (84).

23. Ensemble de séparation de l'une des revendications 20 à 22, dans lequel au moins une partie du trou traversant (46, 42B, 46C, 46D, 146, 146A, 246, 246A, 346, 446, 546) est orientée le long de l'axe longitudinal (L) du récipient de collecte (82) dans la première position initiale, et où au moins une partie du trou traversant (46, 42B, 46C, 46D, 146, 146A, 246, 246A, 346, 446, 546) est orientée perpendiculairement à l'axe longitudinal (L) du récipient de collecte (82) dans la deuxième position d'étanchéité.

24. Séparateur mécanique (40, 40B, 240) de l'une des revendications 1 à 19, dans lequel au moins une partie du flotteur (42, 42B, 242) comprend un périmètre extérieur circulaire ayant une section transversale incurvée perpendiculaire au trou traversant (46, 46B, 246), dans lequel le périmètre extérieur du flotteur (42, 42B, 242) forme un joint d'étanchéité continu avec la surface intérieure du récipient de collecte (82) dans la deuxième position d'étanchéité.
